(19) Europäisches Patentamt
European Patent Office
Office européen des brevets

(11) Veröffentlichungsnummer: **0 245 860**
**A2**

# (12) EUROPÄISCHE PATENTANMELDUNG

(21) Anmeldenummer: **87107010.8**

(22) Anmeldetag: **14.05.87**

(51) Int. Cl.4: **C07D 471/04**, A01N 43/90, //C07D491/147,C07D471/22,(C-07D471/04,231:00,221:00),(C07-D491/147,307:00,231:00,221:00-),(C07D471/22,231:00,221:00,2-09:00,209:00)

(30) Priorität: **16.05.86 DE 3616849**

(43) Veröffentlichungstag der Anmeldung:
**19.11.87 Patentblatt 87/47**

(84) Benannte Vertragsstaaten:
**AT BE CH DE ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Nordhoff, Erhard, Dr.**
**Markelstrasse 45**
**D-1000 Berlin 41(DE)**
Erfinder: **Franke, Wilfried, Dr.**
**Karl-Stieler-Strasse 2 b**
**D-1000 Berlin 41(DE)**
Erfinder: **Arndt, Friedrich, Dr.**
**Mühlenfeldstrasse 10**
**D-1000 Berlin 28(DE)**
Erfinder: **Kötter, Clemens, Dr.**
**Laurinsteig 26**
**D-1000 Berlin 28(DE)**

## (54) Pyrazolo [3,4-b]pyridinderivate, Verfahren und Zwischenstufen zu ihrer Herstellung und ihre Verwendung als Mittel mit herbizider Wirkung.

(57) Die Erfindung betrifft neue Pyrazolo[3,4-b]pyridinderivate der allgemeinen Formel I

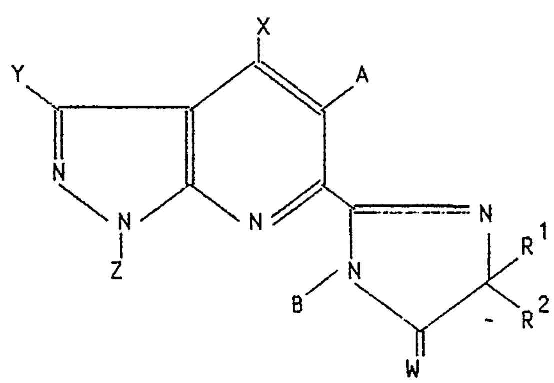

(I) ,

in der $R^1$, $R^2$, A, B, W, X, Y und Z die in der Beschreibung genannten Bedeutung haben, Verfahren und Zwischenstufen zu ihrer Herstellung und ihre Verwendung als Mittel mit herbizider Wirkung.

EP 0 245 860 A2

## Pyrazolo[3,4-b]pyridinderivate, Verfahren und Zwischenstufen zu ihrer Herstellung und ihre Verwendung als Mittel mit herbizider Wirkung

Die Erfindung betrifft neue Pyrazolo[3,4-b]pyridinderivate, Verfahren und Zwischenstufen zu ihrer Herstellung und ihrer Verwendung als Mittel mit herbizider Wirkung.

Es ist bereits bekannt, daß Imidazolin-Derivate herbizide Eigenschaften besitzen [DE-OS 28 33 274 und DE-OS 31 21 736). Häufig ist jedoch die Herbizidwirkung der bekannten Verbindungen nicht ausreichend, beziehungsweise es treten bei entsprechender Herbizidwirkung Selektivitätsprobleme in landwirtschaftlichen Hauptkulturen auf.

Aufgabe der vorliegenden Anmeldung ist die Bereitstellung von neuen Verbindungen, die diese Nachteile nicht aufweisen und in ihren biologischen Eigenschaften den bisher bekannten Verbindungen überlegen sind.

Es wurde, daß Pyrazolo[3,4-b]pyridinderivate der allgemeinen Formel I

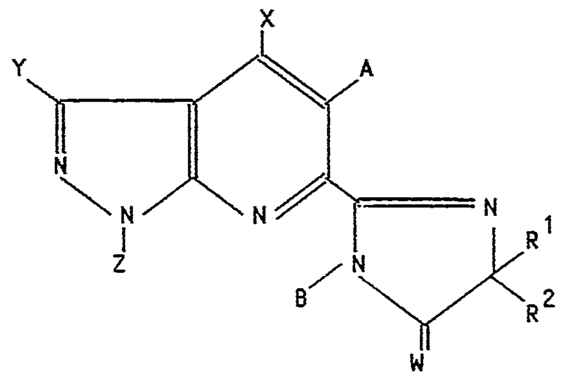

(I) ,

in der

$R^1$ einen $C_1$-$C_4$-Alkylrest,

$R^2$ einen $C_1$-$C_6$-Alkylrest oder einen $C_3$-$C_6$-Cycloalkylrest oder

$R^1$ und $R^2$ gemeinsam mit dem benachbarten Kohlenstoffatom einen gegebenenfalls durch Methyl substituierten $C_3$-$C_6$-Cycloalkylrest,

A eine der Gruppen $CH_2OH$, $COOR^3$ oder $CONR^4R^5$,

$R^3$ ein Wasserstoffatom, einen gegebenenfalls durch Hydroxy, Halogen, $C_1$-$C_4$-Alkoxy, $C_1$-$C_4$-Alkylthio, $C_3$-$C_6$-Cycloalkyl, Benzyl, Furyl, Tetrahydrofuryl, Phenyl, Halogenphenyl, $C_1$-$C_4$-Alkylphenyl, $C_1$-$C_4$-Alkoxyphenyl, Nitrophenyl, Cyano, Carboxyl, $C_1$-$C_4$-Alkoxycarbonyl, $C_1$-$C_4$-Alkylamino oder Di-$C_1$-$C_4$-alkylamino substituierten $C_1$-$C_{12}$-Alkylrest, der gegebenenfalls durch ein oder mehrere Sauerstoff-oder Schwefelatome unterbrochen ist, einen gegebenenfalls durch $C_1$-$C_3$-Alkoxy, Phenyl oder Halogen substituierten $C_3$-$C_8$-Alkenylrest, einen gegebenenfalls durch $C_1$-$C_3$-Alkoxy, Phenyl oder Halogen substituierten $C_3$-$C_8$-Alkinylrest, einen gegebenenfalls durch Methyl substituierten $C_3$-$C_6$-Cycloalkylrest, einen $C_1$-$C_4$-Alkylideniminorest oder ein Kation aus der Gruppe der Alkalimetalle, Erdalkalimetalle, Mangan, Kupfer, Eisen, Zink, Ammonium und organische Ammoniumverbindungen,

$R^4$ und $R^5$ unabhängig voneinander ein Wasserstoffatom, eine Hydroxygruppe, einen gegebenenfalls ein- oder mehrfach durch Hydroxy, Halogen, $C_1$-$C_3$-Alkoxy oder $C_1$-$C_3$-Alkylthio substituierten $C_1$-$C_4$-Alkylrest, einen $C_3$-Alkenylrest oder einem $C_3$-Alkinylrest und für den Fall, daß $R^4$ ein Wasserstoffatom ist, $R^5$ auch einem Amino-, Dimethylamino, Acetylamino-oder Anilinorest,

B ein Wasserstoffatom und für den Fall, daß A = $COOR^3$, wobei aber $R^3$ kein Wasserstoffatom oder ein salzbildendes Kation bedeutet, W = Sauerstoff und die Reste X, Y und Z keinen Alkylamino-, Hydroxy-oder Hydroxyalkylrest darstellen, auch die Gruppen $COR^6$ oder $SO_2R^7$,

$R^6$ einen $C_1$-$C_8$-Alkylrest, einen $C_1$-$C_8$-Alkoxyrest, einen $C_1$-$C_8$-Alkylthiorest, einen Di-$C_1$-$C_8$-Alkylaminorest, einen $C_1$-$C_6$-Halogenalkylrest, einen gegebenenfalls ein-oder mehrfach durch Halogen, Nitro, $C_1$-$C_4$-Alkyl oder $C_1$-$C_4$-Alkoxy substituierten Phenylrest oder einen gegebenenfalls ein-oder mehrfach durch Halogen, Nitro, $C_1$-$C_4$-Alkyl oder $C_1$-$C_4$-Alkoxy substituierten Phenoxyrest,

$R^7$ einen $C_1$-$C_8$-Alkylrest, einen Trifluormethylrest, einen Tri chlormethylrest oder einen gegebenenfalls ein- oder mehrfach durch Halogen, Nitro, $C_1$-$C_4$-Alkyl oder $C_1$-$C_4$-Alkoxy substituierten Phenylrest,

W ein Sauerstoff-oder Schwefelatom,

X ein Wasserstoffatom, eine Nitrogruppe, einen $C_1$-$C_3$-Alkylrest, einen $C_1$-$C_3$-Halogenalkylrest oder einen $C_1$-

$C_3$-Alkoxyrest,

Y ein Wasserstoffatom, ein Halogenatom, eine Cyanogruppe, eine Nitrogruppe, einen $C_1$-$C_3$-Alkoxycarbonylrest, einen Pyridylrest, einen N-Methylpyrrolylrest, einen Aminorest, einen $C_1$-$C_4$-Alkylaminorest, einen Di-$C_1$-$C_4$-Alkylaminorest, einen $C_3$-$C_6$-Cycloalkylaminorest, einen Pyrrolidinylrest, einen Piperidinylrest, einen Aziridinylrest, einen Morpholinylrest, einen $C_1$-$C_4$-Alkylthiorest, einen $C_3$-$C_6$-Cycloalkylthiorest, einen $C_1$-$C_4$-Alkoxyrest, einen $C_3$-$C_6$-Cycloalkoxyrest, einen $C_1$-$C_4$-Alkylsulfonylrest, einen Tolylsulfonylrest, einen $C_3$-$C_6$-Cycloalkylrest, einen gegebenenfalls ein-oder mehrfach durch Halogen, $C_1$-$C_3$-Alkoxy, $C_1$-$C_3$-Alkylthio, Hydroxy, $C_3$-$C_6$-Cycloalkyl, Cyano, Phenyl, Furyl, Tetrahydrofuryl substituierten $C_1$-$C_8$-Alkylrest, der gegebenenfalls durch ein oder mehrere Sauerstoff-oder Schwefelatome unterbrochen ist, einen gegebenenfalls ein-oder mehrfach durch Halogen, $C_1$-$C_3$-Alkyl, $C_1$-$C_3$-Alkoxy, Halogenmethyl oder Nitro substituierten Phenylrest, einen gegebenenfalls ein-oder mehrfach durch Halogen, $C_1$-$C_3$-Alkyl, $C_1$-$C_3$-Alkoxy, Halogenmethyl oder Nitro substituierten Phenoxyrest, einen $C_3$-$C_6$-Alkenylrest oder einen $C_3$-$C_6$-Alkinylrest und

Z ein Wasserstoffatom, einen $C_1$-$C_3$-Alkoxycarbonylrest, einen $C_1$-$C_8$-Alkoxysulfinylrest, einen Phenylsulfonylrest, einen $C_1$-$C_8$-Alkylsulfonylrest, einen $C_1$-$C_3$-Alkylphenylsulfonylrest, einen Carbamoylrest, einen Thiocarbamoylrest, einen Cyanorest, einen Pyridylrest, einen Naphthylrest, einen Acetylrest, einen Benzoylrest, einen Di-$C_1$-$C_6$-alkylcarbamoylrest, einen $C_3$-$C_6$-Cycloalkylcarbamoylrest, einen Amidinorest, einen $C_3$-$C_6$-Cycloalkylrest, einen $C_3$-$C_6$-Alkenylrest, einen $C_3$-$C_6$-Alkinylrest, einen gegebenenfalls ein-oder mehrfach, gleich oder verschieden durch Halogen, Hydroxy, $C_1$-$C_6$-Alkoxy, $C_3$-$C_6$-Cycloalkyl, $C_3$-$C_6$-Cycloalkyloxy, $C_1$-$C_4$-Alkylthio, $C_3$-$C_6$-Cycloalkylthio, Amino, $C_1$-$C_3$-Alkylamino, Di-$C_1$-$C_3$-alkylamino, $C_3$-$C_6$-Cycloalkylamino, Phenyl oder Cyano substituierten $C_1$-$C_8$-Alkylrest, der gegebenenfalls durch ein oder mehrere Sauerstoff-oder Schwefelatome unterbrochen ist, einen gegebenenfalls ein-oder mehrfach, gleich oder verschieden durch Halogen, $C_1$-$C_3$-Alkyl, $C_1$-$C_3$-Alkoxy, Hydroxy, Cyano oder Nitro substituierten Pyrimidinylrest oder einen gegebenenfalls ein-oder mehrfach, gleich oder verschieden durch Halogen, $C_1$-$C_3$-Alkyl, $C_1$-$C_3$-Alkoxy, Hydroxy, Cyano oder Nitro substituierten Phenylrest bedeuten, sowie für den Fall, daß W ein Sauerstoffatom und A die Gruppe $COOR^3$, aber $R^3$ nicht ein ungesättigter Alkylrest ist, die N-Oxide der Verbindungen, für den Fall, daß $R^1$ und $R^2$ nicht die gleiche Bedeutung haben, die optischen Isomeren der Verbindungen und für den Fall, daß $R^3$ kein salzbildendes Kation darstellt, die Säureadditionssalze der Verbindungen, eine interessante herbizide Wirkung zeigen.

Die Bezeichnung "Alkalimetall" umfaßt Natrium, Kalium und Lithium.

Die Bezeichnung "Organische Ammoniumverbindungen" definiert eine Gruppe mit einem positiv geladenen Stickstoffatom, welches mit 1 bis 4 aliphatischen Gruppen verbunden ist, wobei jede 1 bis 12 Kohlenstoffatome enthalten kann, wie zum Beispiel Monoalkylammonium, Dialkylammonium, Trialkylammonium, Tetra-alkylammonium, Cycloalkylammonium, Piperidinium, Morpholinium und Pyrrolidinium.

Die Bezeichnung "Halogen" umfaßt Fluor, Chlor, Brom und Jod.

Überraschenderweise zeigen die erfindungsgemäßen Verbindungen bessere selektive, beziehungsweise Wirkungs-Eigenschaften als die aus dem Stand der Technik bekannten Verbindungen. Von den neuen Verbindungen zeichnen sich durch ihre herbizide Wirkung insbesondere diejenigen aus, bei denen in der allgemeinen Formel I

$R^1$ einen $C_1$-$C_4$-Alkylrest,

$R^2$ einen $C_1$-$C_6$-Alkylrest,

A die Gruppen $COOR^3$ oder $CONR^4R^5$,

$R^3$ ein Wasserstoffatom, einen $C_1$-$C_4$-Alkylrest, einen $C_3$-$C_6$-Alkenylrest, $C_3$-$C_6$-Alkinylrest, einen Tetrahydrofurfurylrest oder eine organische Ammoniumverbindung,

$R^4$ ein Wasserstoffatom oder einen $C_1$-$C_4$-Alkylrest,

$R^5$ ein Wasserstoffatom oder einen $C_1$-$C_4$-Alkylrest,

B ein Wasserstoffatom,

W ein Sauerstoffatom,

X ein Wasserstoffatom,

Y ein Wasserstoffatom, einen $C_1$-$C_4$-Alkylrest, einen Halogen-$C_1$-$C_4$-alkylrest, einen $C_1$-$C_4$-Alkoxyrest oder einen $C_1$-$C_4$-Alkylthiorest und

Z einen Methylrest oder einen Phenylrest bedeuten.

Die erfindungsgemäßen Verbindungen der allgemeinen Formel I lassen sich zum Beispiel herstellen, indem man

A) falls A = COOH und B = Wasserstoff bedeuten, eine Verbindung der allgemeinen Formel II

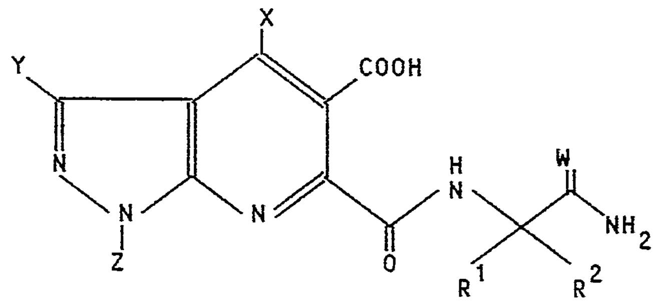

(II) ,

in der $R^1$, $R^2$, W, X, Y und Z die im Anspruch 1 angegebenen Bedeutungen haben, unter alkalischen Bedingungen cyclisiert,

B) falls A = $COOR^3$, $R^3$ aber nicht für ein Wasserstoffatom oder ein salzbildendes Kation steht, und B = Wasserstoff bedeuten, eine Verbindung der allgemeinen Formel III

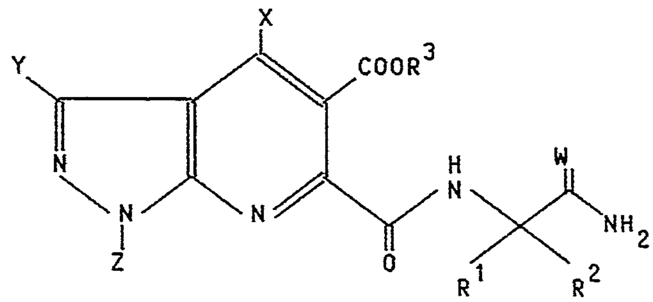

(III) ,

in der $R^1$, $R^2$, $R^3$, W, X, Y und Z die im Anspruch 1 angegebenen Bedeutungen haben, $R^3$ aber nicht für ein Wasserstoffatom oder ein salzbildendes Kation steht, mit einem Gemisch aus Phosphoroxychlorid und Phosphorpentachlorid umsetzt und aus den erhaltenen Hydrochloridsalzen die Ester durch Neutralisation mit einer geeigneten Base in Freiheit setzt,

C) falls A = $COOR^3$, $R^3$ aber nicht für ein Wasserstoffatom oder ein salzbildendes Kation steht, B = Wasserstoff und W = Sauerstoff bedeuten, eine Verbindung der allgemeinen Formel IV

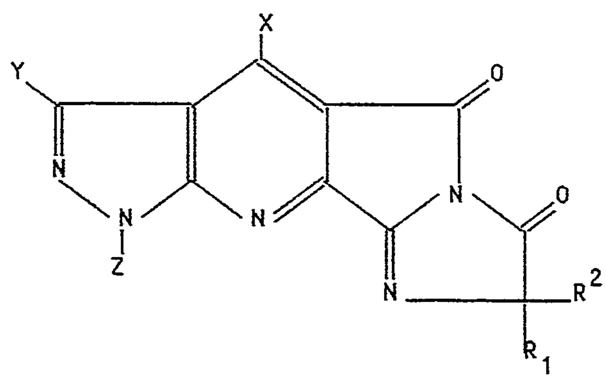

(IV) ,

in der $R^1$, $R^2$, X, Y und Z die im Anspruch 1 angegebenen Bedeutungen haben, mit einem Alkohol der allgemeinen Formel

$R^3$ -OH

und einem entsprechenden Alkalimetallalkoxid der allgemeinen Formel

$R^3$ -OM,

in denen $R^3$ die im Anspruch 1 angegebenen Bedeutungen hat, aber nicht für ein Wasserstoffatom oder ein salzbildendes Kation steht, und M ein Alkalimetall darstellt, gegebenenfalls in Gegenwart eines Lösungsmittels umsetzt,

D) falls A = $CONR^4R^5$, B = Wasserstoff und W = Sauerstoff bedeuten, eine Verbindung der allgemeinen Formel IV

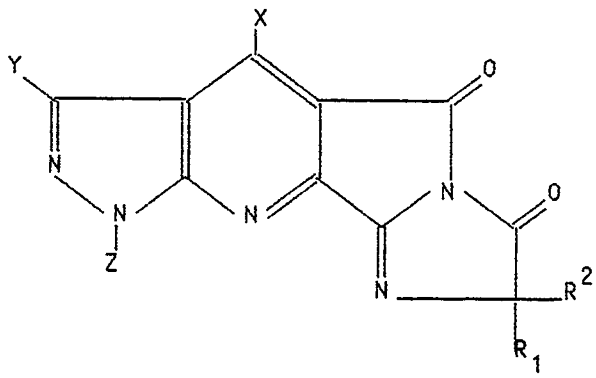

(IV) ,

in der $R^1$, $R^2$, X, Y und Z die im Anspruch 1 angegebenen Bedeutungen haben, mit einem Amin der allgemeinen Formel

$HNR^4R^5$,

in der $R^4$ und $R^5$ die im Anspruch 1 angegebenen Bedeutungen haben, gegebenenfalls unter Verwendung eines Lösungsmittels umsetzt,

E) falls A = $CH_2OH$, B = Wasserstoff und W = Sauerstoff bedeuten, eine Verbindung der allgemeinen Formel IV

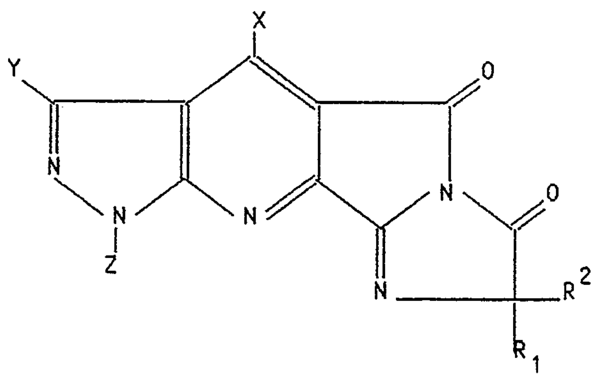

(IV) ,

in der $R^1$, $R^2$, X, Y und Z die im Anspruch 1 angegebenen Bedeutungen haben, mit einem Reduktionsmittel, vorzugsweise Natriumborhydrid, zur Reaktion bringt,

F) falls A = $COOR^3$, $R^3$ = ein salzbildendes Kation und B = Wasserstoff bedeuten, eine Verbindung der allgemeinen Formel Ia

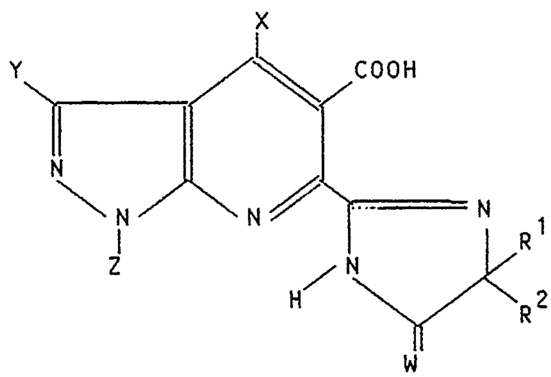

(I a) ,

in der $R^1$, $R^2$, W, X, Y und Z die im Anspruch 1 angegebenen Bedeutungen haben, mit einem Äquivalent einer Base als Salzbildner umsetzt,

G) falls A = $COOR^3$, $R^3$ aber nicht für ein Wasserstoffatom oder ein salzbildendes Kation steht, B = $COR^6$ oder $SO_2R^7$ und W = Sauerstoff bedeuten, eine Verbindung der allgemeinen Formel Ib

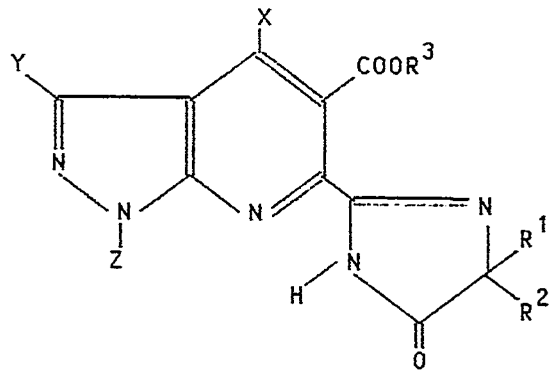

(I b) ,

in der $R^1$, $R^2$, $R^3$, X, Y und Z die im Anspruch 1 angegebenen Bedeutungen haben, $R^3$ aber nicht für ein Wasserstoffatom oder ein salzbildendes Kation steht, mit einem Acylhalogenid der allgemeinen Formel
$R^6$-CO - Hal
oder einem Säureanhydrid der allgemeinen Formel
$R^6$-CO - O - CO - $R^6$
oder einem Sulfonylhalogenid der allgemeinen Formel
$R^7$-$SO_2$-Hal,
bei denen $R^6$ und $R^7$ die im Anspruch 1 angegebenen Bedeutungen haben und Hal für ein Halogenatom steht, gegebenenfalls in Gegenwart eines Lösungsmittels und eines säurebindenden Mittels umsetzt oder

H) falls A = $COOR^3$, $R^3$ aber nicht für ein Wasserstoffatom oder ein salzbildendes Kation steht, B = Wasserstoff und W = Sauerstoff bedeuten, eine Verbindung der allgemeinen Formel Ib

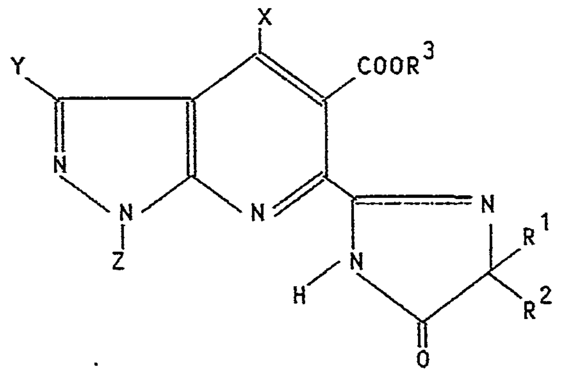

(I b) ,

in der $R^1$, $R^2$, $R^3$, X, Y und Z die im Anspruch 1 angegebenen Bedeutungen haben, $R^3$ aber nicht für ein Wasserstoffatom oder ein salzbildendes Kation steht, mit mindestens einem Äquivalent Halogenwasserstoffsäure in einem organischen Lösungsmittel zu einem Halogenwasserstoffsäure-Additionssalz umsetzt.

Die Verfahrensvariante A) wird zweckmäßigerweise so durchgeführt, daß man das Ausgangsmaterial der allgemeinen Formel II über längere Zeit, wie 0,5 bis 20 Stunden, bei einer Temperatur im Bereich von 20 bis 100°C mit einer wäßrigen oder auch wäßrig-alkoholischen Alkalimetallhydroxidlösung, wie Natrium-oder Kaliumhydroxidlösung, behandelt, wobei die Menge des Hydroxids, bezogen auf das Ausgangsmaterial der allgemeinen Formel II, 2 bis 10 Moläquivalente beträgt. Anschließend wird das Reaktionsgemisch abgekühlt und mit einer starken Mineralsäure, wie Schwefel-oder Salzsäure, angesäuert.

Die als Ausgangsmaterial verwendeten Amide der allgemeinen Formel II lassen sich entsprechend dem folgenden Formelschema herstellen:

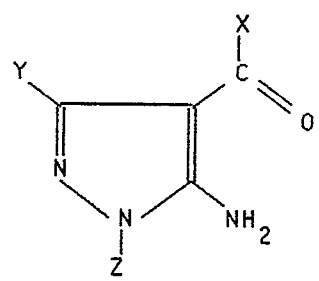

(V)

+

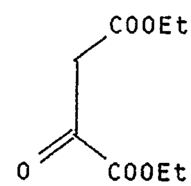

(VI)

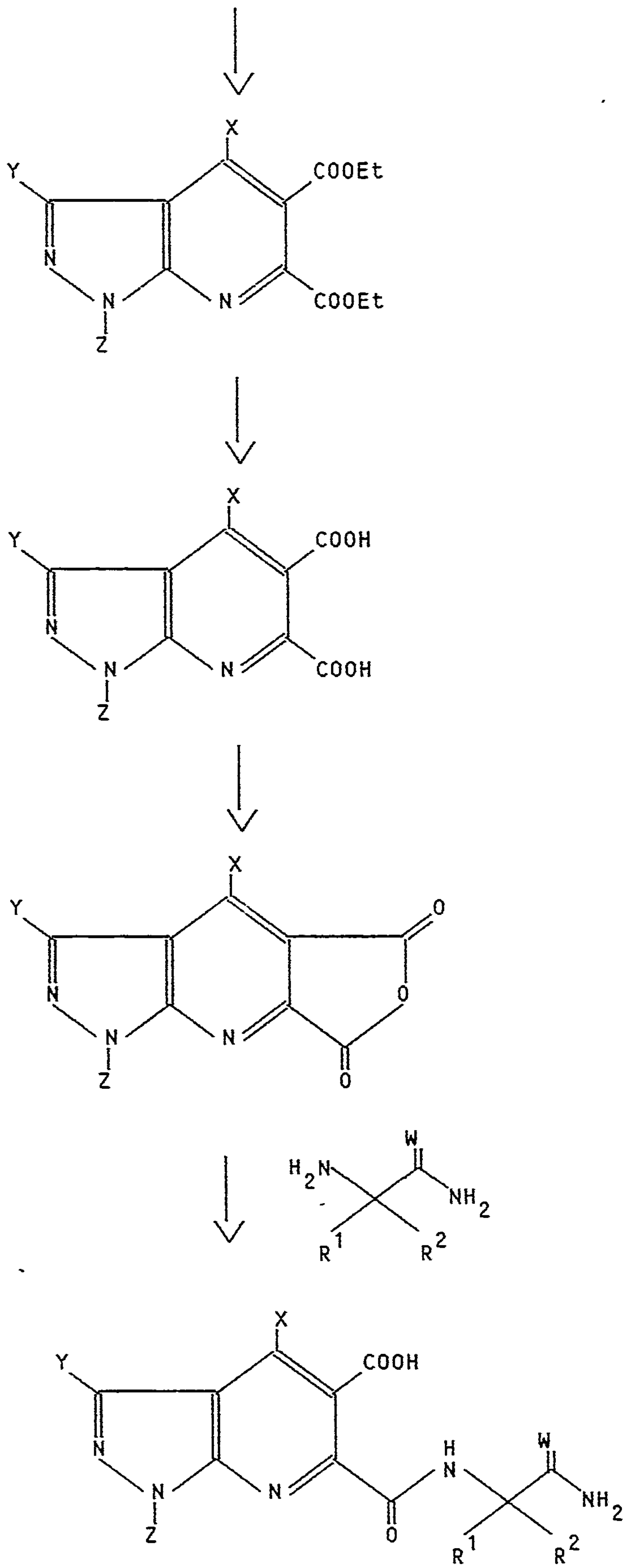

(VII)

(VIII)

(IX)

(X)

(II) ,

Verbindungen der allgemeinen Formel V,. in der X, Y und Z die unter der allgemeinen Formel I definierten Bedeutungen haben (die Verbindungen sind teilweise literaturbekannt oder lassen sich nach analogen, literaturbekannten Verfahren herstellen), kann man durch Umsetzung mit Oxalessigsäurediethylestern der Formel VI in die Pyrazolo[3,4-b]pyridindicarbonsäurediethylester der allgemeinen Formel VII überführen.

Die Diester der allgemeinen Formel VII können zu den Pyrazolo[3,4-b]pyridindicarbonsäuren der allgemeinen Formel VIII hydrolisiert werden, und zwar durch Umsetzung mit einer starken Base, wie Kaliumhydroxid oder Natiumhydroxid.

Die Säureanhydride der allgemeinen Formel IX können anschließend durch Behandlung der Pyrazolo[3,4-b]pyridindicarbonsäuren der allgemeinen Formel VIII mit beispielsweise Essigsäureanhydrid erhalten werden.

Durch Umsetzung mit einem Aminosäureamid der allgemeinen Formel X in dem $R^1$ und $R^2$ die unter der allgemeinen Formel I definierten Bedeutung haben, werden Verbindung der allgemeinen Formel II erhalten.

Die Herstellung von Verbindungen der allgemeinen Formel II erfolgt in Gegenwart eines organischen Lösungsmittels, wie zum Beispiel Diethylether, Tetrahydrofuran, Acetonitril oder Essigester, oder in einem halogenierten Lösungsmittel, wie zum Beispiel Methylenchlorid oder Chloroform. Die Reaktion findet in einem Temperaturbereich von 20° bis 100°C statt. Bei der Reaktion in geringen Mengen anfallende isomere Pyrazolo[3,4-b]pyridincarbonsäurederivate lassen sich leicht durch Umkristallisation oder Chromatographie entfernen.

Viele der Pyrazolo[3,4-b]pyridindicarbonsäurederivate der allgemeinen Formel VII können auch nach dem folgenden Formelschema erhalten werden:

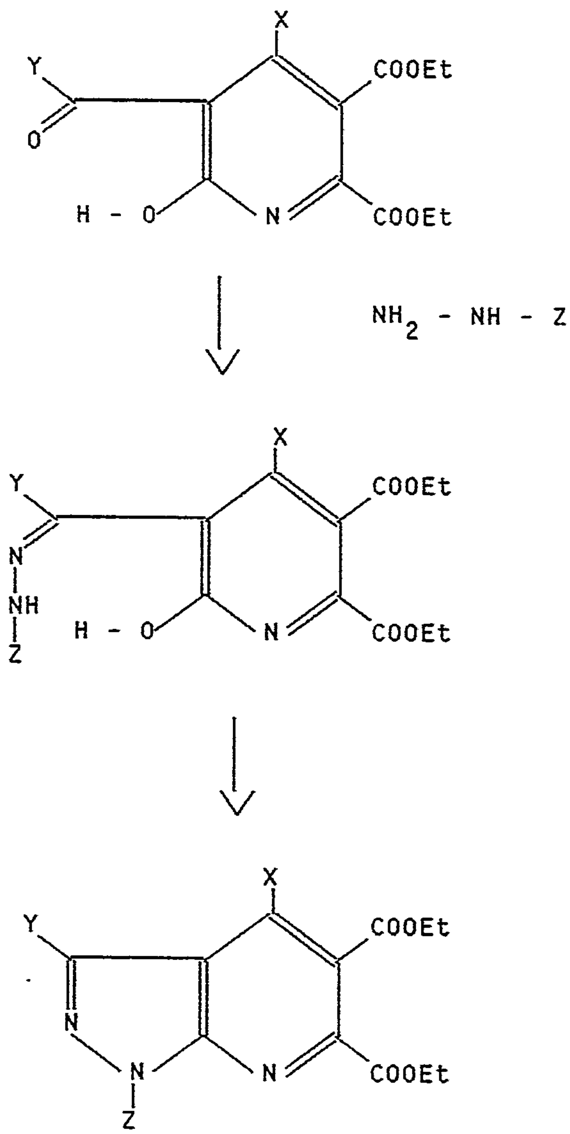


(XI)

(XII)

(XIII)

(VII)

Man bringt die zweckentsprechend subtituierten Pyridindicarbonsäureester der allgemeinen Formel XI, in der X und Y die unter der allgemeinen Formel I definierten Bedeutungen haben, mit einem Hydrazin der allgemeinen Formel XII, in der Z die unter der allgemeinen Formel I definierte Bedeutung hat, in einem geeigneten Lösungsmittel, wie zum Beispiel $C_1$-$C_4$-Alkoholen, Methylenchlorid, Chloroform, Acetonitril, Dimethylsulfoxid, Dimethylformamid, Toluol, Benzol, halogenierte Benzole, Dioxan, Tetrahydrofuran, Diethylether oder Wasser, in einem Temperaturbereich von 0°C bis zur Siedetemperatur des Lösungsmittels zur Reaktion.

Für Reaktionen, bei denen Salze der Hydrazine der allgemeinen Formel XII, zum Beispiel die Hydrochloride, eingesetzt werden, können gegebenenfalls Säurefänger, wie Natriumacetat, Natriumhydrogencarbonat, Natriumcarbonat, und Amine, wie Triethylamin, zugesetzt werden.

Pyridindicarbonsäurederivate der allgemeinen Formel XI sind teilweise literaturbekannt oder lassen sich nach analogen literaturbekannten Verfahren darstellen.

Je nach Ausgangsmaterial und Reaktionsbedingungen können auch die als Zwischenprodukt gebildeten Hydrazone der allgemeinen Formel XIII isoliert werden.

Die Hydrazone der allgemeinen Formel XIII werden anschließend zu den Pyrazolo[3,4-b]-pyridindicarbonsaurederivaten der allgemeinen Formel VII cyclisiert. Zur Cyclisierung können Reagenzien, wie zum Beispiel Acetanhydrid, Trifluoressigsäureanhydrid, Thionylchlorid, Sulfurylchlorid, Oxalylchlorid, Phosphoroxychlorid, konz. Schwefelsäure, Eisessig oder Ameisensäure, gegebenenfalls unter Zugabe eines Losungsmittels, wie Chloroform, Methylenchlorid, Dimethylsulfoxid, Dimethylformamid, Toluol oder Benzol, und einer Base, wie Pyridin oder Triethylamin, in einem Temperaturbereich von 0°C bis zur Siedetemperatur des Reaktionsgemisches verwendet werden.

Pyrazolo[3,4-b]pyridindicarbonsäurederivate der allgemeinen Formel VII, in der Y für Fluor, Chlor, Brom, Jod, Hydroxy oder Cyano steht, werden nach nach dem folgenden Formelschema erhalten:

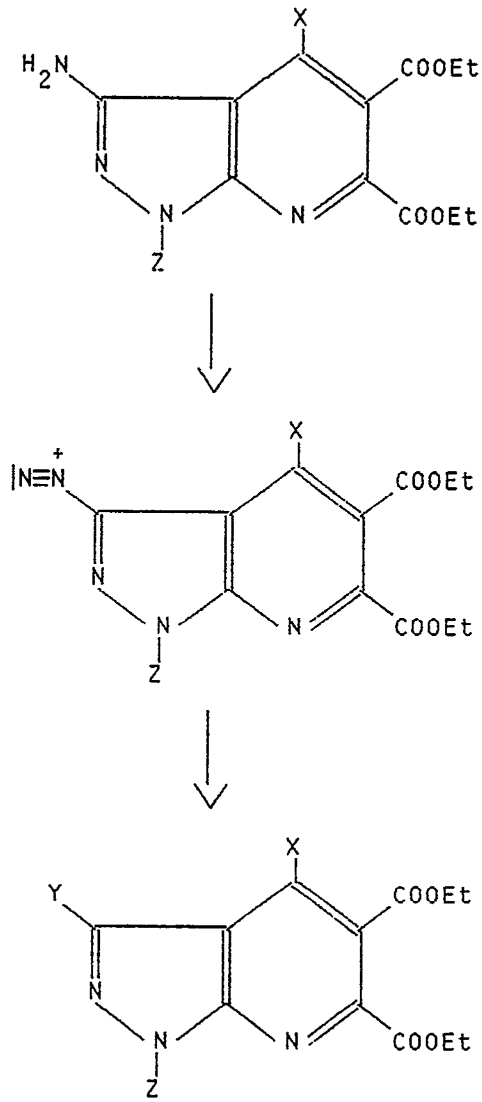

(XIV)

(XV)

(VII)

3-Amino-pyrazolo[3,4-b]pyridin-dicarbonsäurediethylester der allgemeinen Formel XIV, bei denen X und Z die unter der allgemeinen Formel I angegebenen Bedeutungen haben, lassen sich in die entsprechenden Diazoniumsalze der allgemeinen Formel XV überführen. Diese werden anschließend durch eine Sandmeyer-Reaktion in Pyrazolo[3,4-b]pyridindicarbonsäurediethylester der Formel VII umgewandelt, bei denen Y für Chlor, Brom, Jod oder Cyano steht. Durch Verkochen der Diazoniumsalze in den entsprechenden Lösungsmitteln lassen sich zum Beispiel auch Hydroxy und Fluor als Substituenten einfügen.

Die Darstellung von Diazoniumsalzen und deren weitere Umsetzungen erfolgt analog zu literaturbekannten Verfahren.

Die Verfahrensvariante B) wird zweckmaßigerweise so durchgeführt, daß man das Ausgangsmaterial der allgemeinen Formel III direkt mit einem Gemisch aus Phosphoroxychlorid und Phosphorpentachlorid bei Raumtemperatur zur Reaktion bringt.

Die Reaktion kann auch in Gegenwart eines unter den Reakionsbedingungen inerten Lösungsmittels, wie zum Beispiel Toluol, Xylol oder Chloroform, in einem Temperaturbereich von 0°C bis 100°C durchgeführt werden. Die freien Basen werden aus den dabei anfallenden Hydrochloriden nach an sich üblichen Methoden, zum Beispiel durch Umsetzung mit Natriumcarbonat oder Natriumhydrogencarbonat, isoliert.

Das Ausgangsmaterial der allgemeinen Formel III wird aus Verbindungen der allgemeinen Formel II durch Veresterung nach an sich üblichen Methoden mit den entsprechenden Alkoholen der allgemeinen Formel $R^3$-OH erhalten.

Die Verfahrensvariante C wird zweckmäßigerweise so durchgeführt, daß man das Ausgangsmaterial der allgemeinen Formel IV mit einer Mischung aus Alkalimetallhydrid, wie Natriumhydrid, und dem entsprechenden Alkohol der Formel $R^3$-OH im Überschuß 0,5 bis 5 Stunden bei einer Temperatur im Bereich von Raumtemperatur bis zur Siedetemperatur des Reaktionsgemisches umsetzt. Der eingesetzt Alkohol dient bei dieser Umsetzung sowohl als Reaktand als auch als Lösungsmittel. Es können jedoch auch zusätzliche Lösungsmittel wie zum Beispiel Tetrahydrofuran, Dioxan oder andere nicht protische Lösungsmittel eingesetzt werden.

Die Verfahrensvariante D wird zweckmäßigerweise so durchgeführt, daß man das Ausgangsmaterial der allgemeinen Formel IV mit einem primären oder sekundären Amin der allgemeinen Formel $HNR^4R^5$, wobei $R^4$ und $R^5$ die unter der allgemeinen Formel 1 angegebenen Bedeutung haben, in einem Temperaturbereich von 20° bis 100°C, gegebenenfalls unter Verwendung eines Lösungsmittels, wie zum Beispiel Dimethylformamid, Tetrahydrofuran oder Dioxan, zur Reaktion bringt.

Die Verfahrensvariante E wird zweckmäßigerweise so durchgeführt, daß man das Ausgangsmaterial der allgemeinen Formel IV mit einem Reduktionsmittel, wie Natriumborhydrid, in einem Temperaturbereich von -10°C bis 30°C in einem geeigneten Lösungsmittel oder Lösungsmittelgemisch, wie zum Beispiel Ethanol/Tetrahydrofuran, zur Reaktion bringt.

Das Ausgangsmaterial der Formel IV läßt sich aus Verbindungen der allgemeinen Formel I c, in der A = COOH, B = Wasserstoff und W = Sauerstoff bedeuten, durch Umsetzung mit Dicyclohexylcarbodiimid darstellen.

Die Umsetzung erfolgt in inerten Lösungsmitteln, wie zum Beispiel chlorierten Kohlenwasserstoffen, unter Verwendung einer äquimolaren Menge des Carbodiimids in einem Temperaturbereich von 20°C bis 60°C entsprechend dem folgenden Reaktionsschema:

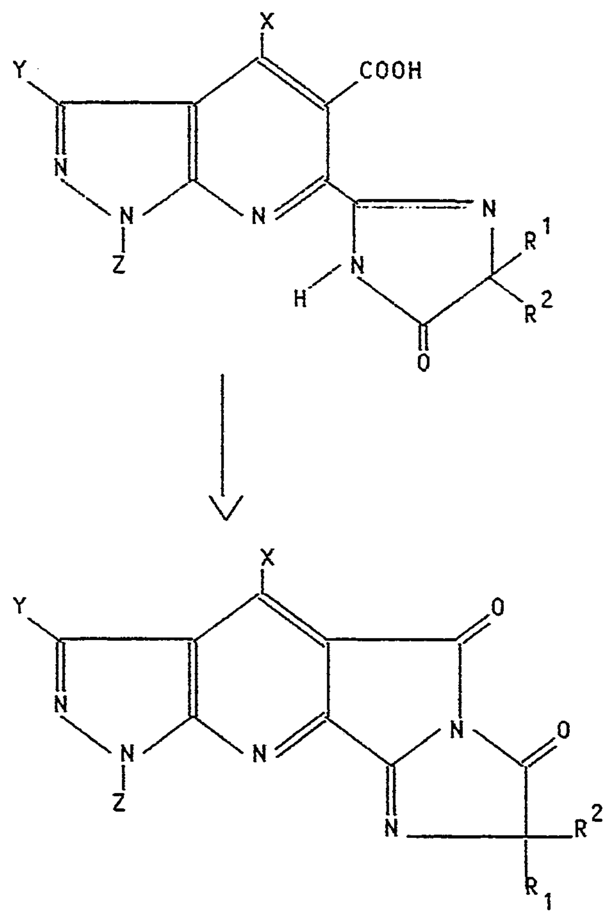

(I c) ,

(IV)

Die Verfahrensvariante F wird zweckmäßigerweise so durchgeführt, daß man das Ausgangsmaterial der Formel I, in der A für COOH und B für Wasserstoff steht, mit einem Äquivalent einer Base als Salzbildner, wie zum Beispiel Alkalimetall-oder Erdalkalimetallhydroxid, Ammoniumhydroxid, der einem Alkylamin, wie zum Beispiel Isopropylamin, in einem geeigneten Lösungsmittel bei Raumtemperatur zur Reaktion bringt.

Die Verfahrensvariante G wird so durchgeführt, daß man das entsprechend Verfahrensvariante B und C erhaltene Ausgangsmaterial der allgemeinen Formel I mit einer überschüssigen Menge eines Acylhalogenids der allgemeinen Formel $R^6$-CO-Hal, eines Säureanhydrids der allgemeinen Formel $R^6$-CO-O-CO-$R^6$ oder eines Sulfonylhalogenids der Formel $R^7$-$SO_2$-Hal, wobei $R^6$ und $R^7$ die unter der allgemeinen Formel I angegebenen Bedeutungen haben und Hal für ein Halogenatom steht, gegebenenfalls in Gegenwart eines Lösungsmittels, und eines säurebindenden Mittels, wie Pyridin, bei einer Temperatur zwischen 50° und 130°C umsetzt.

Die nach den oben genannten Verfahrensvarianten hergestellten erfindungsgemäßen Verbindungen können nach üblichen Verfahren aus dem Reaktionsgemisch isoliert werden, beispielsweise indem man das Reaktionsgemisch in Eiswasser gibt und das Produkt durch Extraktion mit einem geeigneten Lösungsmittel oder durch Filtration abtrennt.

Die erfindungsgemäßen Verbindungen stellen in der Regel kristalline oder zähflüssige Substanzen dar, die zum Teil gut löslich in halogenierten Kohlenwasserstoffen, wie Chloroform, Sulfoxiden, wie Dimethylsulfoxid, oder Estern, wie Essigester, sind.

Die vorliegenden erfindungsgemäßen Verbindungen weisen eine ausgezeichnete herbizide Wirksamkeit gegen wirtschaftlich wichtige mono-und dikotyle Unkräuter auf. Auch schwer bekämpfbare perennierende Unkräuter werden durch die Wirkstoffe gut erfaßt. Dabei ist es gleichbültig, ob die Substanzen in Vorsaat-, Vorauflauf-oder Nachauflaufspritzung ausgebracht werden. Werden die erfindungsgemäßen Verbindungen vor dem Keimen auf die Erdoberfläche appliziert, so wird das Auflaufen der Keimlinge nicht vollständig verhindert. Die Unkräuter wachsen bis zum Keimblattstadium heran, stellen jedoch dan ihr Wachstum ein und sterben schließlich ab.

Die Verbindungen werden durch Wurzeln und Blätter aufgenommen und in der Pflanze verteilt unter Anreicherung in meristematischen Regionen. In behandelten Pflanzen wird das Wachstum bald nach der Spritzung unterbunden. Chlorose tritt auf. In perennierenden Unkräutern werden die Verbindungen in unterirdische Teile transportiert, so daß der Wiederaustrieb verhindert wird.

Die erfindungsgemäßen Wirkstoffe können zum Beispiel gegen die folgenden Pflanzengattungen eingesetzt werden:

Dikotyle Unkräuter der Gattungen Abutilon, Chrysanthemum, Brassica, Helianthus, Mentha, Sinapis, Lepidium, Galium, Stellaria, Anthemis, Chenopodium, Atriplex, Senecio, Portulaca, Ipomea, Matricaria, Galinsoga, Urtica, Amaranthus, Convolvulus, Polygonum, Sesbania, Ambrosia, Cirsium, Carduus, Sonchus, Solanum, Rorippa, Lamium, Veronica, Datura, Viola, Centaurea und Galeopsis.

Monokotyle Unkräuter der Gattungen Avena, Alopecurus, Echinochloa, Seteria, Panicum, Digitaria, Poa, Eleusine, Brachiaria, Lolium, Bromus, Cyperus, Agropyron, Sagittaria, Cynodon, Monochoria, Fimbristylis, Eleocharis, Ischaemum und Apera.

Die Verbindungen können in landwirtschaftlichen Hauptkulturen wie Baumwolle, Soja und anderen Leguminosen, Reis, Mais, Weizen, Gerste, Hafer, Sorghum, Zuckerrohr und Ananas angewendet werden.

Die Verwendung der erfindungsgemäßen Wirkstoffe ist keineswegs auf die genannten Unkrautgattungen und Kulturpflanzen beschränkt, sondern erstreckt sich in gleicher Weise auch auf andere Pflanzen.

Die Verbindungen eignen sich in hoheren Aufwandmengen auch zur Totalunkrautbekämpfung, zum Beispiel auf Industrie-und Gleisanlagen und auf Wegen und Platzen. Ebenso können die Verbindungen zur Unkrautbekämpfung in Dauerkulturen, wie zum Beispiel Forst-, Ziergehölz-, Obst-, Wein-, Citrus-, Nuß-, Bananen-, Kaffee-, Tee-, Gummi-, Ölpalm-, Kakao-, Beerenfrucht-, und Hopfenanlagen, eingesetzt werden.

Darüber hinaus weisen die Verbindungen in entsprechend niederen Konzentrationen wachstumsregulatorische Eigenschaften bei Kulturpflanzen auf, indem sie regulierend in den pflanzeneigenen Stoffwechsel eingreifen, so daß sie zum Beispiel verwendet werden können zur generellen Steuerung des vegetativen und generativen Wachstums.

Die angewandte Wirkstoffmenge kann in einem größeren Bereich schwanken. Sie hängt im wesentlichen von der Art des gewünschten Effekts ab. Im allgemeinen liegen die Aufwandmengen zwischen 0,01 und 5 kg Wirkstoff pro Hektar Bodenfläche, vorzugsweise zum Beispiel bei der Unkrautbekämpfung zwischen 0,1 und 0,5 kg Wirkstoff pro Hektar.

Die erfindungsgemäßen Verbindungen können entweder allein, in Mischung miteinander oder mit anderen Wirkstoffen angewendet werden. Gegebenenfalls können andere Pflanzenschutz-oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden. Beispielsweise eignen sich als herbizid wirksame Mischungspartner diejenigen Wirkstoffe, die in Weed Abstracts, Vol. 34, No.3, 1985, unter dem Titel "List of common names and abbreviations employed for currently used herbicides and plant-growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acylphosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zugbereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier-und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl-oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

1.) 20 Gewichtsprozent Wirkstoff
68 Gewichtsprozent Kaolin
10 Gewichtsprozent Calciumsalz der Ligninsulfonsäure
2 Gewichtsprozent Dialkylnaphthalinsulfonat

2.) 40 Gewichtsprozent Wirkstoff
25 Gewichtsprozent Kaolin
25 Gewichtsprozent kolloidale Kieselsäure
8 Gewichtsprozent Calciumsalz der Ligninsulfonsäure
2 Gewichtsprozent Natriumsalz des N-Methyl-N-oleyltaurins

B) **Wasserlösliches Pulver**

10 Gewichtsprozent Wirkstoff
75 Gewichtsprozent Harnstoff
9 Gewichtsprozent Mischpolymerisationsprodukt aus Ethylenoxid und Propylenoxid
1 Gewichtsprozent Dialkylnaphthalinsulfonat
5 Gewichtsprozent Natriumsalz der Ethylendiamintetraessigsäure

C) **Emulsionskonzentrat**

20 Gewichtsprozent Wirkstoff
75 Gewichtsprozent Isophoron
2 Gewichtsprozent ethoxyliertes Rizinusöl
3 Gewichtsprozent Calciumsalz der Dodecylphenylsulfonsäure

Die nachfolgenden Beispiele erläutern die Herstellung der erfindungsgemäßen Verbindungen:

**Beispiel 1** (Verfahren A)

6-(4-Isopropyl-4-methyl-5-oxo-2-imidazolin-2-yl)-1-methyl-1H-pyrazolo[3,4-b]pyridin-5-carbonsäure, Monohydrat

7,5 g (0,022 mol) 6-[N-(1-Carbamoyl-1,2-dimethylpropyl)-carbamoyl]-1-methyl-1H-pyrazolo[3,4-b]-pyridin-5-carbonsäure und 60 ml 2,6 N Natronlauge wurden bei Raumtemperatur zusammengegeben und anschließend 2 Stunden bei 70°C gerührt. Unter Eiskühlung wurde das Reaktionsgemisch mit konz. Salzsäure bis auf pH 1 angesäuert und das auskristallisierte Produkt abfiltriert.

Ausbeute: 4,1 g = 57,8 % der Theorie

Fp.: 258°C Zersetzung (Monohydrat)

Analyse: für $C_{15}H_{17}N_5O_3$ x $H_2O$ MG: 333,33

berechnet: 54,05 % C 5,75 % H 21,01 % N

gefunden: 54,06 % C 5,37 % H 20,86 % N

Das Ausgangsmaterial wurde wie folgt hergestellt:

a) Herstellung der Verbindungen der allgemeinen Formel VII

$a_1$) 1-Methyl-1H-pyrazolo[3,4-b]pyridin-5,6-dicarbonsäurediethylester

1,2 g (9,6 mmol) 5-Amino-4-formyl-1-methyl-pyrazol, 1,8 g (9,6 mmol) Oxalessigsäurediethylester, 1 ml Piperidin und 1 g Molekularsieb (4 A, pulverisiert) wurden 6 Stunden bei 80°C gerührt, anschließend filtriert und eingeengt. Der Ruckstand wurde über Keiselgel mit Hexan/Essigester als Laufmittel chromatographiert.

Ausbeute: 1,1 g = 41,3 % der Theorie

Fp.: 74-76°C

Analyse: für $C_{13}H_{15}N_3O_4$ MG: 277,28

berechnet: 56,31 % C 5,45 % H 15,15 % N

gefunden: 56,47 % C 5,75 % H 14,85 % N

$a_2$) 1,3-Dimethyl-1H-pyrazolo[3,4-b]pyridin-5,6-dicarbonsaurediethylester, Semihydrat

2,0 g (0,007 mol) 6-Hydroxy-5-(1-methylhydrazono-ethyl)-2,3-pyridindicarbonsäure-diethylester wurden in 30 ml Eisessig suspendiert und über Nacht bei Raumtemperatur gerührt. Das Reaktionsgemisch wurde in 150 ml Eiswasser gegeben und mit Methylenchlorid extrahiert. Die organische Phase wurde über Magnesiumsulfat getrocknet, filtriert und eingeengt.

Ausbeute: 0,3 g = 14,3 % der Theorie

Fp.: 116-118°C

Analyse: für $C_{14}H_{17}N_3O_4$ MG: 300,331

berechnet: 55,98 % C 6,06 % H 13,99 % N

gefunden: 56,06 % C 6,34 % H 13,66 % N

Das Ausgangsmaterial von $a_2$) wurde wie folgt hergestellt:

6-Hydroxy-5-(1-methylhydrazono-ethyl)-2,3-pyridindicarbonsäurediethylester

8,4 g (0,03 mol) 5-Acetyl-6-hydroxy-pyridin-5,6-dicarbonsäurediethylester wurden in 100 ml Methanol gelöst und mit 1,6 ml (0,03 mol) Methylhydrazin tropfenweise versetzt. Das Reaktionsgemisch wurde für 2 Stunden auf 50°C erwärmt und das auskristallisierte Produkt nach dem Abkühlen abfiltriert und aus Ethanol umkristallisiert.

Ausbeute: 6,0 g = 67,4 % der Theorie

Fp.: 154-157°C

Analyse: für $C_{14}H_{19}N_3O_5$ MG: 309,327

berechnet: 54,36 % C 6,19 % H 13,58 % N

gefunden: 54,37 % C 5,96 % H 13,36 % N

Die folgenden Verbindungen der allgemeinen Formel VII wurden ebenfalls nach den obigen Verfahren hergestellt:

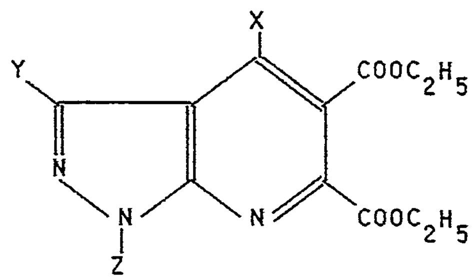

(VII)

| X | Y | Z | Fp. |
|---|---|---|---|
| H | H | $C_6H_5$ | 86-87°C |
| H | $CH_3$ | $C_6H_5$ | 119°C |
| H | H | H | 110-112°C |
| H | $CH_3$ | H | 173-175°C |
| H | $C_2H_5$ | H | 139-140°C |
| H | $C_6H_5$ | H | 136-137°C |
| H | $C_2H_5$ | $CH_3$ | 114°C |
| H | $OC_2H_5$ | $CH_3$ | 105-106°C |
| H | $CH_3$ | $C_2H_5$ | 74-75°C |
| H | $C_2H_5$ | $C_2H_5$ | 64°C |
| H | $CH_3$ | $(CH_2)_2CH_3$ | 65-66°C |
| H | $CH_3$ | $CH(CH_3)_2$ | Öl |
| H | $CH_3$ | $C(CH_3)_2$ | 84-85°C |
| H | $C_2H_5$ | $C_6H_5$ | 112°C |
| H | H | $CH_2C_6H_5$ | 206°C Zers. |
| H | $CH_2Cl$ | $CH_3$ | 78-81°C |
| H | $SCH_3$ | $CH_3$ | 85-86°C |
| H | $OCH_3$ | $CH_3$ | 94-95°C |
| H | $CH_3$ | $CH_2C{\equiv}CH$ | 79-80°C |
| H | $CH_3$ | $CH_2CH{=}CH_2$ | 59-62°C |
| H | $NH_2$ | H | 149-152°C |
| H | $CH_2OCH_3$ | $CH_3$ | 55-57°C |

b) Herstellung der Verbindung der allgemeinen Formel VIII 1-Methyl-1H-pyrazolo[3,4-b]pyridin-5,6-dicarbonsäure

20 g (72,13 mmol) 1-Methyl-1H-pyrazolo[3,4-b]pyridin-5,6-dicarbonsäurediethylester wurden zu einer Lösung aus 8,7 g (216,4 mmol) Natriumhydroxid in 100 ml Wasser gegeben und die Reaktionsmischung 20 Stunden bei 60°C gerührt. Anschließend wurde das Reaktionsgemisch unter Eiskühlung mit konzentrierter Salzsäure bis auf pH 1 angesäuert und das auskristallisierte Produkt abfiltriert.

Ausbeute: 14,0 g = 87,8 % der Theorie

Fp.: 225°C Zersetzung

Analyse: für $C_9H_7N_3O_4$ MG: 221,17
berechnet: 48,88 % C 3,19 % H 19,00 % N
gefunden: 48,88 % C 3,24 % H 19,10 % N

Die folgenden Verbindungen der allgemeinen Formel VIII wurden ebenfalls nach dem obigen Verfahren hergestellt:

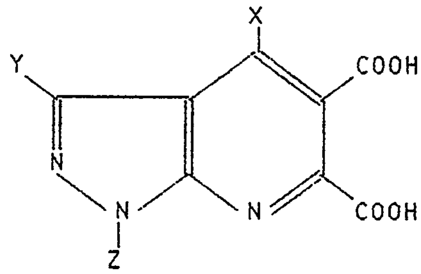

(VIII) ,

| X | Y | Z | Fp. | Bemerkungen |
|---|---|---|---|---|
| H | H | $C_6H_5$ | 212°C Zers. | |
| H | $CH_3$ | $CH_3$ | 216°C Zers. | |
| H | $CH_3$ | $C_6H_5$ | 198°C | |
| H | $CH_3$ | H | >250°C | x 2 $H_2O$ |
| H | $C_2H_5$ | H | >250°C | |
| H | $C_2H_5$ | $CH_3$ | 232°C Zers. | |
| H | $OC_2H_5$ | $CH_3$ | >250°C | x $H_2O$ |
| H | $CH_3$ | $C_2H_5$ | 207°C Zers. | |
| H | $C_2H_5$ | $C_2H_5$ | 206°C Zers. | |
| H | H | $(CH_2)_2CH_3$ | 201°C Zers. | |
| H | $CH_3$ | $(CH_2)_2CH_3$ | 193°C | |
| H | H | $CH(CH_3)_2$ | 216°C | |
| H | $CH_3$ | $CH(CH_3)_2$ | 208°C Zers. | |
| H | $C_2H_5$ | $CH(CH_3)_2$ | 185°C | |
| H | H | $C(CH_3)_3$ | 205°C Zers. | |
| H | $CH_3$ | $C(CH_3)_3$ | 205°C Zers. | x $H_2O$ |
| H | $C_2H_5$ | $C_6H_5$ | 201°C Zers. | |
| H | H | $CH_2C_6H_5$ | 206°C Zers. | x 1/2 $H_2O$ |
| H | $CH_2OH$ | $CH_3$ | 243°C Zers. | x 2 $H_2O$ |
| H | $OCH_3$ | $CH_3$ | >250°C | |
| H | $SCH_3$ | $CH_3$ | 198°C Zers. | |
| H | $CH_3$ | $CH_2CH{=}CH_2$ | >250°C | |
| H | $CH_3$ | $CH_2C{\equiv}CH$ | 176°C Zers. | |
| H | $CH_2OCH_3$ | $CH_3$ | 208°C Zers. | |

c) Herstellung der Verbindungen der allgemeinen Formel IX 1-Methyl-1H-pyrazolo[3,4-b]pyridin-5,6-dicarbonsäureanhydrid

10,0 g (45,21 mmol) 1-Methyl-1H-pyrazolo[3,4-b]pyridin-5,6-dicarbonsäure und 50 ml Acetanhydrid wurden 2 Stunden unter Rückfluß erwärmt, das Reaktionsgemisch anschließend auf 0°C abgekühlt und das auskristallisierte Produkt abfiltriert.

Ausbeute: 6,4 g = 69,7 % der Theorie

Fp.: 196-197°C

Analyse: für $C_9H_5N_3O_3$ MG: 203,16

berechnet: 53,21 % C 2,48 % H 20,68 % N

gefunden: 53,16 % C 2,84 % H 20,57 % N

Die folgenden Verbindungen der allgemeinen Formel IX wurden ebenfalls nach dem obigen Verfahren hergestellt:

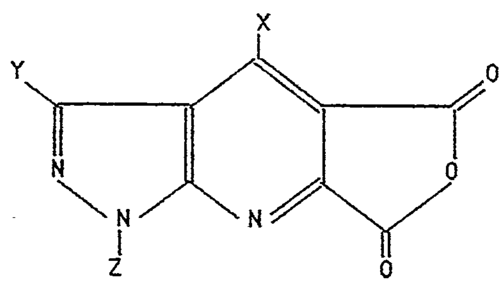

(IX)

| X | Y | Z | Fp. |
|---|---|---|---|
| H | H | $C_6H_5$ | 219-220°C |
| H | $CH_3$ | $CH_3$ | 247-249°C |
| H | $CH_3$ | $C_6H_5$ | 232-233°C |

d) Herstellung der Verbindungen der allgemeinen Formel II 6-[N-(1-Carbamoyl-1,2-dimethylpropyl)-carbamoyl]-1-methyl-1H-pyrazolo[3,4-b]pyridin-5-carbonsäure

6,25 g (30,76 mmol) 1-Methyl-1H-pyrazolo[3,4-b]pyridin-5,6-dicarbonsäureanhydrid wurden in 60 ml Tetrahydrofuran suspendiert und bei einer Temperatur von 60°C durch Zugabe von etwa 10 ml Acetonitril in Lösung gebracht. Anschließend wurde eine Lösung von 4,41 g (33,84 mmol) 2-Methylvalin-amid in 10 ml Acetonitril zugetropft und 2 Stunden bei 60°C gerührt. Das auskristallisierte Produkt wurde abfiltriert und mit Ether und Pentan gewaschen.

Ausbeute: 9 g = 87,8 % der Theorie

Fp.: 211°C Zersetzung

Analyse: für $C_{15}H_{11}N_5O_4$ MG: 333,35

berechnet: 54,05 % C 5,75 % H 21,00 % N

gefunden: 54,10 % C 6,18 % H 20,47 % N

Die folgenden Verbindungen der allgemeinen Formel II wurden ebenfalls nach dem obigen Verfahren hergestellt:

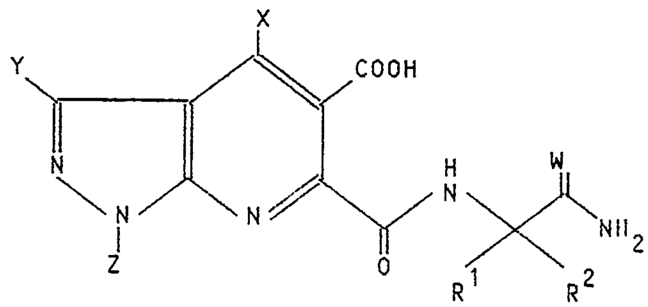

(II) ,

| X | Y | Z | W | $R^1$ | $R^2$ | Fp. | Bemerkungen |
|---|---|---|---|---|---|---|---|
| H | H | $C_6H_5$ | O | $CH_3$ | $CH(CH_3)_2$ | | |
| H | $CH_3$ | $CH_3$ | O | $CH_3$ | $CH(CH_3)_2$ | 165°C Zers. | x $H_2O$ |
| H | $CH_3$ | $C_6H_5$ | O | $CH_3$ | $CH(CH_3)_2$ | 222°C Zers. | |
| H | H | $CH(CH_3)_2$ | O | $CH_3$ | $CH(CH_3)_2$ | 198°C | |
| H | $C_2H_5$ | $C_6H_5$ | O | $CH_3$ | $CH(CH_3)_2$ | 221°C Zers. | |
| H | H | $CH_2C_6H_5$ | O | $CH_3$ | $CH(CH_3)_2$ | 185°C | |
| H | $SCH_3$ | $CH_3$ | O | $CH_3$ | $CH(CH_3)_2$ | 173°C Zers. | |
| H | $CH_3$ | $CH_2CH{=}CH_2$ | O | $CH_3$ | $CH(CH_3)_2$ | 71-79°C | |
| H | $CH_3$ | H | O | $CH_3$ | $CH(CH_3)_2$ | 196°C | |

**Beispiel 2** (Verfahren F)

6-(4-Isopropyl-4-methyl-5-oxo-2-imidazolin-2-yl)-1-methyl-1H-pyrazolo[3,4-b]pyridin-5-carbonsäure, Isopropyl-ammoniumsalz, Monohydrat

2 g (6,34 mmol) 6-(4-Isopropyl-4-methyl-5-oxo-2-imidazolin-2-yl)-1-methyl-1H-pyrazolo[3,4-b]pyridin-5-carbonsäure und 0,38 g (6,34 mmol) Isopropylamin in 50 ml Methylenchlorid wurden bei Raumtemperatur gerührt und das nach 1 Stunde auskristallisierte Produkte abfiltriert.

Ausbeute: 2,3 g = 97 % der Theorie

Fp.: 240°C Zersetzung (Monohydrat)

Analyse: für $C_{15}H_{17}N_5O_3$ x $H_2NCH(CH_3)_2$ x $H_2O$

berechnet: 55,01 % C 7,19 % H 21,41 % N

gefunden: 55,77 % C 6,50 % H 21,74 % N

**Beispiel 3** (Verfahren C)

6-(4-Isopropyl-4-methyl-5-oxo-2-imidazolin-2-yl)-1-methyl-1H-pyrazolo[3,4-b]pyridin-5-carbonsäuremethylester

3 g (10,09 mmol) 6-Isopropyl-3,6-dimethyl-imidazol[1',2':1,2]pyrrolo[3,4-b]pyrazolo[4,3-e]pyridin-7,9-(3H,6H)-dion wurden in 50 ml Methanol gelöst und zusammen mit katalytischen Mengen Natriumhydrid 3 Stunden unter Rückfluß erhitzt, über Nacht bei Raumtemperatur stehengelassen und das auskristallisierte Produkt abfiltriert.

Ausbeute: 3,0 g = 90,3 % der Theorie

Fp.: 212°C Zersetzung

Analyse: für $C_{16}H_{19}N_5O_3$ MG: 329,36
berechnet: 58,35 % C 5,81 % H 21,26 % N
gefunden: 58,13 % C 5,83 % H 21,02 % N

Das Ausgangsmaterial wurde wie folgt hergestellt:

6-Isopropyl-3,6-dimethyl-imidazo[1',2':1,2]pyrrolo[3,4-b]pyrazolo[4,3-e]pyridin-7,9(3H,6H)-dion

12,7 g (40,28 mmol) 6-(4-Isopropyl-4-methyl-5-oxo-2-imidazolin-2-yl)-1-methyl-1H-pyrazolo[3,4-b]-pyridin-5-carbonsäure und 9,14 g (44,30 mmol) Dicyclohexylcarbodiimid in 120 ml Methylenchlorid wurden 2 Tage bei Raumtemperatur gerührt. Der ausgefallene Harnstoff wurde abfiltriert, gründlich mit Methylenchlorid gewaschen, das Filtrat eingeengt und das Produkt erhalten.

Ausbeute: 11,8 g = 99 % der Theorie
Fp.:
Analyse: für $C_{15}H_{15}N_5O_2$ MG: 297,30
berechnet: 61,40 % C 5,76 % H 22,45 % N
gefunden: 60,60 % C 5,09 % H 23,40 % N

Die folgenden Verbindungen der allgemeinen Formel IV wurden ebenfalls nach dem obigen Verfahren hergestellt

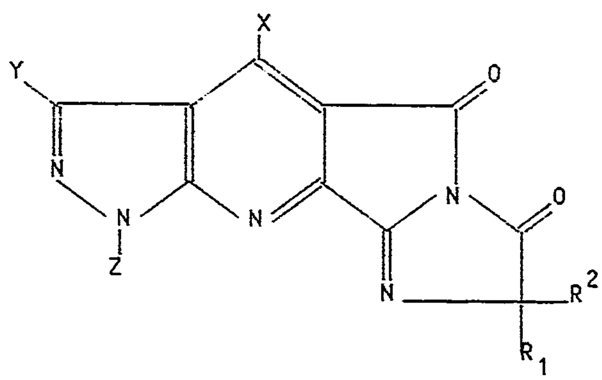

(IV)

| X | Y | Z | $R^1$ | $R^2$ | Fp. |
|---|---|---|---|---|---|
| H | H | $C_6H_5$ | $CH_3$ | $CH(CH_3)_2$ | 175°C Zers. |
| H | $CH_3$ | $CH(CH_3)_2$ | $CH_3$ | $CH(CH_3)_2$ | 190-194°C |
| H | $CH_2Cl$ | $CH_3$ | $CH_3$ | $CH(CH_3)_2$ | 130°C Zers. |

**Beispiel 4** (Verfahren D)

6-(4-Isopropyl-4-methyl-4-oxo-2-imidazolin-2-yl)-1-methyl-1H-pyrazolo[3,4-b]pyridin-5-carbonsäureamid

3 g (10,09 mmol) 6-Isopropyl-3,6-dimethyl-imidazol[1',2':1,2]pyrrolo[3,4-b]pyrazolo[4,3-e]pyridin-7,9-(3H,6H)-dion wurde in 50 ml Methylenchlorid gelöst, 1 Stunde Ammoniak-Gas durch die Lösung geleitet und anschließend die ausgefallenen Kristalle abgesaugt.

Ausbeute: 2,2 g = 66 % der Theorie
Fp.: 213-214°C
Analyse: für $C_{15}H_{18}N_6O_3$ MG: 330,35
berechnet: 54,53 % C 5,49 % H 25,44 % N
gefunden: 54,83 % C 5,18 % H 25,31 % N

In analoger Weise können auch die folgende Verbindungen der allgemeinen Formel I hergestellt werden:

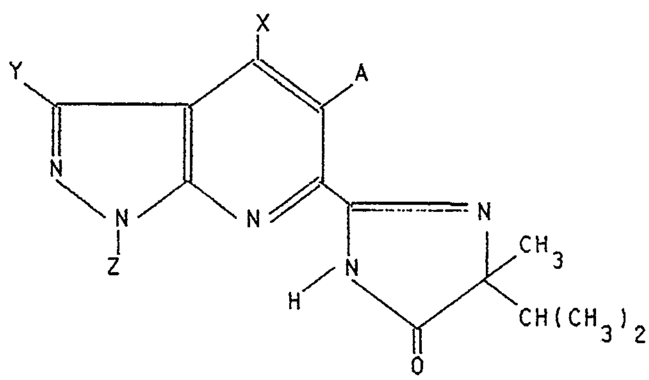

| Beispiel Nr. | X | Y | Z | A | Fp. |
|---|---|---|---|---|---|
| 5 | H | H | $C_6H_5$ | COOH x 1/2 $H_2O$ | 180°C Zers. |
| 6 | H | $CH_3$ | $CH_3$ | COOH | 248°C Zers. |
| 7 | H | H | $C_6H_5$ | $CONH_2$ | 229-231°C |

| Beispiel Nr. | X | Y | Z | A | Fp. |
|---|---|---|---|---|---|
| 8 | H | H | $C_6H_5$ | $COOCH_3$ | 176-178°C |
| 9 | H | H | $C_6H_5$ | $COOCH_2$-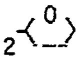 | 180°C Zers. |
| 10 | H | H | $C_6H_5$ | $COOCH_2C{\equiv}CH$ | 71-73°C |
| 11 | H | H | $C_6H_5$ | COOH<br>x $H_2NCH(CH_3)_2$<br>x $2H_2O$ | 198°C |
| 12 | H | H | $CH_3$ | $COOCH_2$-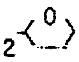 | 203°C |
| 13 | H | H | $CH_3$ | $COOCH_2C{\equiv}CH$ | 204°C |
| 14 | H | $CH_3$ | $CH_3$ | $COOCH_3$ | 209-210°C |
| 15 | H | $CH_3$ | $C_6H_5$ | $COOCH_3$ | 188-190°C |
| 16 | H | $CH_3$ | $C_2H_5$ | COOH<br>x $H_2O$ | 220-222°C |
| 17 | H | $CH_3$ | $C(CH_3)_3$ | COOH<br>x $H_2O$ | 240°C Zers. |
| 18 | H | $CH_3$ | $C(CH_3)_3$ | COOH<br>x $H_2NCH(CH_3)_2$ | 250°C Zers. |
| 19 | H | H | $CH_2C_6H_5$ | COOH<br>x $H_2NCH(CH_3)_2$<br>x 2 $H_2O$ | 105°C Zers. |
| 20 | H | H | $CH_2C_6H_5$ | COOH<br>x 2 $H_2O$ | 221°C Zers. |
| 21 | H | $CH_3$ | $C_2H_5$ | COOH<br>x $NH_3$ | 238°C Zers. |
| 22 | H | H | $(CH_2)_2CH_3$ | COOH | 194°C Zers. |
| 23 | H | H | $C(CH_3)_3$ | COOH<br>x 2,5 $H_2O$ | 280°C Zers. |
| 24 | H | H | $(CH_2)_3CH_3$ | COOH<br>x 2 $H_2O$ | 265°C Zers. |
| 25 | H | H | $CH_3$ | COOH<br>x $NH_3$ | 258°C Zers. |
| 26 | H | H | $C_2H_5$ | COOH | 248°C Zers. |
| 27 | H | $C_2H_5$ | $CH_3$ | COOH | 197-198°C |

| Beispiel Nr. | X | Y | Z | A | Fp. |
|---|---|---|---|---|---|
| 28 | H | H | $CH(CH_3)_2$ | COOH | 230-231°C |
| 29 | H | $C_2H_5$ | H | COOH | 235°C Zers. |
| | | | | x $H_2O$ | |
| 30 | H | $CH_3$ | $CH(CH_3)_2$ | COOH | 243°C Zers. |
| 31 | H | $CH_3$ | $(CH_2)_2CH_3$ | COOH | 216-218°C |
| 32 | H | $C_2H_5$ | $C_2H_5$ | COOH | 196°C |
| 33 | H | $C_2H_5$ | $CH(CH_3)_2$ | COOH | 213°C |
| | | | | x 3 $H_2O$ | |
| 34 | H | $C_2H_5$ | $C_6H_5$ | COONa | 290°C Zers. |
| | | | | x 2 $H_2O$ | |
| 35 | H | $C_6H_5$ | $CH_3$ | COOH | 246-248°C Zers. |
| 36 | H | $OCH_3$ | $CH_3$ | COOH | 235-236°C |
| 37 | H | $OCH_3$ | $CH_3$ | COOH | 248°C Zers. |
| | | | | x $H_2NCH(CH_3)_2$ | |
| 38 | H | $OCH_3$ | $CH_3$ | $COOCH_3$ | |
| 39 | H | $OC_2H_5$ | $CH_3$ | COOH | >250°C |
| 40 | H | $OC_2H_5$ | $CH_3$ | COOH | 247°C |
| | | | | x $H_2HCH(CH_3)_2$ | |
| 41 | H | $OC_2H_5$ | $CH_3$ | $COOCH_3$ | 180-181°C |
| 42 | H | $SCH_3$ | $CH_3$ | COOH | 255°C Zers. |
| 43 | H | $SCH_3$ | $CH_3$ | COOH | |
| | | | | x $H_2HCH(CH_3)_2$ | |
| 44 | H | $SCH_3$ | $CH_3$ | $COOCH_3$ | |
| 45 | H | CN | $CH_3$ | COOH | 243°C Zers. |
| 46 | H | CN | $CH_3$ | COOH | |
| | | | | x $H_2NCH(CH_3)_2$ | |
| 47 | H | CN | $CH_3$ | $COOCH_3$ | |
| 48 | H | $NHCH_3$ | $CH_3$ | COOH | 232°C |
| 49 | H | $NHCH_3$ | $CH_3$ | COOH | |
| | | | | x $H_2NCH(CH_3)_2$ | |
| 50 | H | $NHCH_3$ | $CH_3$ | $COOCH_3$ | |
| 51 | H | $N(CH_3)_2$ | $CH_3$ | COOH | 209°C |
| 52 | H | $N(CH_3)_2$ | $CH_3$ | COOH | |
| | | | | x $H_2NCH(CH_3)_2$ | |
| 53 | H | $N(CH_3)_2$ | $CH_3$ | $COOCH_3$ | |

| Beispiel Nr. | X | Y | Z | A | Fp. |
|---|---|---|---|---|---|
| 54 | H | $CH_2Cl$ | $CH_3$ | COOH | 201°C Zers. |
| 55 | H | $CH_2Cl$ | $CH_3$ | $COOCH_3$ | |
| 56 | H | $CH_2Cl$ | $CH_3$ | COOH x $H_2NCH(CH_3)_2$ | |
| 57 | H | $CHCl_2$ | $CH_3$ | COOH | |
| 58 | H | $CHCl_2$ | $CH_3$ | COOH x $H_2NCH(CH_3)_2$ | |
| 59 | H | $CHCl_2$ | $CH_3$ | $COOCH_3$ | |
| 60 | H | $CCl_3$ | $CH_3$ | COOH | |
| 61 | H | $CCl_3$ | $CH_3$ | COOH x $H_2HCH(CH_3)_2$ | |
| 62 | H | $CCl_3$ | $CH_3$ | $COOCH_3$ | |
| 63 | H | $CH_2Br$ | $CH_3$ | COOH | 198°C Zers. |
| 64 | H | $CH_2Br$ | $CH_3$ | COOH x $H_2NCH(CH_3)_2$ | |
| 65 | H | $CH_2Br$ | $CH_3$ | $COOCH_3$ | |
| 66 | H | $CF_3$ | $CH_3$ | COOH | 228°C |
| 67 | H | $CF_3$ | $CH_3$. | COOH x $H_2NCH(CH_3)_2$ | |
| 68 | H | $CF_3$ | $CH_3$ | $COOCH_3$ | |
| 69 | H | $CH_2OH$ | $CH_3$ | COOH | 135°C Zers. |
| 70 | H | $CH_2OH$ | $CH_3$ | COOH x $H_2NCH(CH_3)_2$ | |
| 71 | H | $CH_2OH$ | $CH_3$ | $COOCH_3$ | |
| 72 | H | $CH_2OCH_3$ | $CH_3$ | COOH | 226-227°C |
| 73 | H | $CH_2OCH_3$ | $CH_3$ | COOH x $H_2NCH(CH_3)_2$ | |
| 74 | H | $CH_2OCH_3$ | $CH_3$ | $COOCH_3$ | |
| 75 | H | $CH_2OC_2H_5$ | $CH_3$ | COOH | |
| 76 | H | $CH_2OC_2H_5$ | $CH_3$ | COOH x $H_2NCH(CH_3)_2$ | |
| 77 | H | $CH_2OC_2H_5$ | $CH_3$ | $COOCH_3$ | |
| 78 | H | $CH_2SCH_3$ | $CH_3$ | COOH | 209°C |
| 79 | H | $CH_2SCH_3$ | $CH_3$ | COOH x $H_2NCH(CH_3)_2$ | |

| Beispiel Nr. | X | Y | Z | A | Fp. |
|---|---|---|---|---|---|
| 80 | H | $CH_2SCH_3$ | $CH_3$ | $COOCH_3$ | |
| 81 | H | $CH_2CH_2Cl$ | $CH_3$ | COOH | 211°C |
| 82 | H | $CH_2CH_2Cl$ | $CH_3$ | COOH x $H_2NCH(CH_3)_2$ | |
| 83 | H | $CH_2CH_2Cl$ | $CH_3$ | $COOCH_3$ | |
| 84 | H | $CH_3$ | $CH_2CH{=}CH$ | COOH | 197-199°C |
| 85 | H | $CH_3$ | $CH_2CH{=}CH$ | COOH x $H_2NCH(CH_3)_2$ | 179-184°C |
| 86 | H | $CH_3$ | $CH_2CH{=}CH$ | $COOCH_3$ | |
| 87 | H | $CH_3$ | $CH_2C{\equiv}CH$ | COOH | 186°C |
| 88 | H | $CH_3$ | $CH_2C{\equiv}CH$ | COOH x $H_2NCH(CH_3)_2$ | |
| 89 | H | $CH_3$ | $CH_2C{\equiv}CH$ | $COOCH_3$ | |
| 90 | H | Cl | $CH_3$ | COOH | 193°C Zers. |
| 91 | H | Br | $CH_3$ | COOH | 176°C Zers. |

Die nachfolgenden Beispiele erläutern die Anwendungsmöglichkeiten der erfindungsgemäßen Verbindungen:

Beispiel A

Im Gewächshaus wurden die in der Tabelle aufgeführten erfindungsgemäßen Verbindungen in einer Aufwandmenge von 3 kg Wirkstoff/ha emulgiert bzw. suspendiert in 500 Liter Wasser/ha auf Brassica spp. und Solanum spp. als Testpflanzen im Vor-und Nachauflauf-Verfahren gespritzt. 3 Wochen nach der Behandlung wurde das Behandlungsergebnis bonitiert, wobei folgendes Schema verwendet wurde:

0 = keine Wirkung
1 = geringe Wirkung oder schwache Inhibition des Pflanzenwuchses
2 = mittlere Wirkung oder Inhibition des Pflanzenwuchses
3 = totale Wuchsinhibition
4 = totale Vernichtung der Pflanzen

In diesem Test zeigte sich bei beiden Testpflanzen sowohl im Vor-wie auch im Nachauflaufverfahren eine totale Vernichtung der Pflanzen (= 4) bei den Verbindungen der Beispiele 1 bis 9 und 11 bis 29.

Beispiel B

Im Gewächshaus wurden aufgeführten Pflanzen vor dem Auflaufen mit den aufgeführten Verbindungen in einer Aufwandmenge von 0,03 kg Wirkstoff/ha behandelt. Die Verbindungen wurden zu diesem Zweck als Suspensionen mit 500 Liter Wasser/ha gleichmaßig über die Pflanzen versprüht. 3 Wochen nach der Behandlung zeigte die erfindungsgemäße Verbindung eine Selektivität in Soja (Glycine max.), Luzerne (Medicago sativa) und Weizen (Triticum aestivum) bei ausgezeichneter Wirkung gegen das Unkraut. Die Vergleichsmittel waren deutlich weniger selectiv beziehungsweise weniger wirksam.

0 = keine Wirkung
1 = geringe Wirkung oder schwache Inhibition des Pflanzenwuchses
2 = mittlere Wirkung oder Inhibition des Pflanzenwuchses
3 = totale Wuchsinhibition
4 = totale Vernichtung der Pflanzen
Gl = Glycine max.
Me = Medicago sativa
Tr = Triticum aestivum
Br = Brassica sp.
So = Solanum sp.
He = Helianthus sp.
St = Stellaria sp.
Ab = Abutilon sp.
Ma = Matricaria sp.
Di = Digitaria sp.

| Erfindungsgemäße Verbindung | Gl | Me | Tr | Br | So | He | St | Ab | Ma | Di |
|---|---|---|---|---|---|---|---|---|---|---|
| Beispiel 1 | 0 | 0 | 0 | 3 | 3 | 3 | 3 | 3 | 3 | 3 |
| Vergleichsmittel | | | | | | | | | | |
| A | 0 | 2 | 3 | 3 | 3 | 3 | 3 | 3 | 3 | 3 |
| B | 0 | 0 | 0 | 0 | 0 | 0 | 0 | 0 | 0 | 0 |

A = 2-(4-Isopropyl-4-methyl-5-oxo-2-imidazolin-2-yl)-chinolin-3-carbonsäure

B = 2-(4-Isopropyl-4-methyl-5-oxo-2-imidazolin-2-yl)-4(5)-methylbenzoesäure-methylester

Beispiel C

Im Gewächshaus wurden die aufgeführten Pflanzen nach dem Auflaufen mit den aufgeführten Verbindungen in einer Aufwandmenge von 0,03 kg Wirkstoff/ha behandelt. Die Verbindungen wurden zu diesem Zweck als Suspensionen mit 500 Liter Wasser/ha gleichmäßig über die Pflanzen versprüht. 3 Wochen nach der Behandlung zeigte die erfindungsgemäße Verbindung eine Selektivität in Luzerne (Medicago sativa), Weizen (Triticum aestivum) und Soja (Glycine max.) bei ausgezeichneter Wirkung gegen das Unkraut. Die Vergleichsmittel waren deutlich weniger selektiv beziehungsweise weniger wirksam.
0 = keine Wirkung
1 = geringe Wirkung oder schwache Inhibition des Pflanzenwuchses
2 = mittlere Wirkung oder Inhibition des Pflanzenwuchses
3 = totale Wuchsinhibition
4 = totale Vernichtung

Me = Medicago sativa
Tr = Triticum aestivum
Gl = Glycine max.
Br = Brassica sp.
So = Solanum sp.
He = Helianthus sp.
St = Stellaria sp.
Ab = Abutilon sp.
Di = Digitaria sp.

| Erfindungsgemäße Verbindung | Me | Tr | Gl | Br | So | He | St | Ab | Di |
|---|---|---|---|---|---|---|---|---|---|
| Beispiel 1 | 0 | 0 | 0 | 3 | 3 | 3 | 3 | 3 | 3 |
| Vergleichsmittel | | | | | | | | | |
| A | 2 | 3 | 0 | 4 | 4 | 3 | 3 | 3 | 3 |
| B | 0 | 0 | 0 | 0 | 0 | 0 | 0 | 0 | 0 |

A = 2-(4-Isopropyl-4-methyl-5-oxo-2-imidazolin-2-yl)-chinolin-3-carbonsäure

B = 2-(4-Isopropyl-4-methyl-5-oxo-2-imidazolin-2-yl)-4(5)-methylbenzoesäure-methylester

Beispiel D

Im Gewächshaus wurden die in der Tabelle aufgeführten Verbindungen mit der ebenfalls erwähnten Aufwandmenge appliziert. Hierzu wurde der Wirkstoff in Gefäße mit 1500 ml Wasser gegeben. Als Testpflanzenarten wurden Echinochloa crus-galli, Cyperus difformis und Cyperus esculentus im 2-bis 5-Blattstadium verwendet. Drei Wochen nach der Applikation wurde die Schädigung der Pflanzen bonitiert.

In der Tabelle bedeuten:

0 = keine Schädigung
1 = schwache Schädigung
2 = mittlere Schädigung
3 = starke Schädigung
4 = total vernichtet

Ec = Echinochloa crus-galli
Cd = Cyperus difformis
Ce = Cyperus esculentus

| Erfindungsgemäße Verbindungen | ppm | Ec | Cd | Ce |
|---|---|---|---|---|
| Beispiel 1 | 10 | 4 | 4 | 4 |
| Beispiel 16 | 10 | 4 | 3 | 4 |
| Beispiel 22 | 10 | 2 | 3 | 3 |
| Beispiel 23 | 10 | 3 | 3 | 3 |
| Beispiel 24 | 10 | 4 | 4 | 4 |
| Beispiel 25 | 10 | 4 | 4 | 4 |
| Beispiel 26 | 10 | 4 | 4 | 4 |
| Beispiel 40 | 10 | 3 | 4 | - |

Wie die Ergebnisse zeigen, ist die erfindungsgemäße Verbindung stark wirksam gegen wichtige Reisunkräuter.

Beispiel E

Ein Unkrautbestand von Brassica sp., Solanum sp., Helianthus sp., Stellaria media, Abutilon sp., Matricaria chamomilla, Chrysanthemum segetum, Ipomoea sp., Avena fatua, Alopecurus myosuroides und Echinochloa crus-galli und eine Kultur von Soja wurden vor dem Auflaufen mit den aufgeführten Verbindungen in einer Aufwandmenge von 0,3 kg Wirkstoff/ha behandelt. Die Verbindungen wurden zu diesem Zweck als Suspensionen mit 500 Liter Wasser/ha gleichmäßig über den Boden versprüht. Drei Wochen nach der Behandlung wurde das Behandlungsergebnis bonitiert.

Die erfindungsgemäßen Verbindungen der Beispiele 1 bis 6, 12 bis 14, 16 bis 18, 20, 22 bis 27, 29 bis 35, 37 und 39 bis 41 vernichteten den Unkrautbestand völlig ohne Schädigung der Soja-Kultur.

## Ansprüche

1. Pyrazolo[3,4-b]pyridinderivate der allgemeinen Formel I

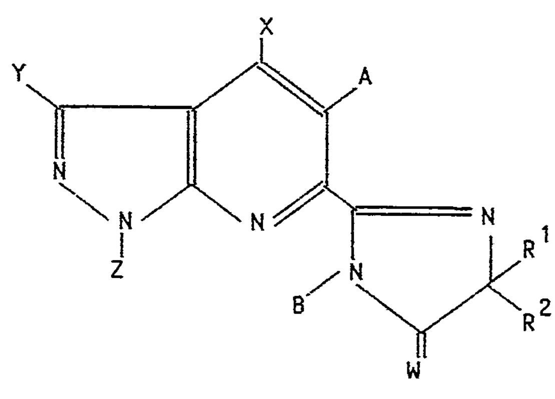

(I) ,

in der

$R^1$ einen $C_1$-$C_4$-Alkylrest,

$R^2$ einen $C_1$-$C_6$-Alkylrest oder einen $C_3$-$C_6$-Cycloalkylrest oder

$R^1$ und $R^2$ gemeinsam mit dem benachbarten Kohlenstoffatom einen gegebenenfalls durch Methyl substituierten $C_3$-$C_6$-Cycloalkylrest,

A eine der Gruppen $CH_2OH$, $COOR^3$ oder $CONR^4R^5$,

$R^3$ ein Wasserstoffatom, einen gegebenenfalls durch Hydroxy, Halogen, $C_1$-$C_4$-Alkoxy, $C_1$-$C_4$-Alkylthio, $C_3$-$C_6$-Cycloalkyl, Benzyl, Furyl, Tetrahydrofuryl, Phenyl, Halogenphenyl, $C_1$-$C_4$-Alkylphenyl, $C_1$-$C_4$-Alkoxyphenyl,

Nitrophenyl, Cyano, Carboxyl, $C_1$-$C_4$-Alkoxycarbonyl, $C_1$-$C_4$-Alkylamino oder Di-$C_1$-$C_4$-alkylamino substituierten $C_1$-$C_{12}$-Alkylrest, der gegebenenfalls durch ein oder mehrere Sauerstoff-oder Schwefelatome unterbrochen ist, einen gegebenenfalls durch $C_1$-$C_3$-Alkoxy, Phenyl oder Halogen substituierten $C_3$-$C_8$-Alkenylrest, einen gegebenenfalls durch $C_1$-$C_3$-Alkoxy, Phenyl oder Halogen substituierten $C_3$-$C_8$-Alkinylrest, einen gegebenenfalls durch Methyl substituierten $C_3$-$C_6$-Cycloalkylrest, einen $C_1$-$C_4$-Alkylideniminorest oder ein Kation aus der Gruppe der Alkalimetalle, Erdalkalimetalle, Mangan, Kupfer, Eisen, Zink, Ammonium und organische Ammoniumverbindungen,

$R^4$ und $R^5$ unabhängig voneinander ein Wasserstoffatom, eine Hydroxygruppe, einen gegebenenfalls ein- oder mehrfach durch Hydroxy, Halogen, $C_1$-$C_3$-Alkoxy oder $C_1$-$C_3$-Alkylthio substituierten $C_1$-$C_4$-Alkylrest, einen $C_3$-Alkenylrest oder einen $C_3$-Alkinylrest und für den Fall, daß $R^4$ ein Wasserstoffatom ist, $R^5$ auch einem Amino-, Dimethylamino, Acetylamino-oder Anilinorest,

B ein Wasserstoffatom und für den Fall, daß A = $COOR^3$, wobei aber $R^3$ kein Wasserstoffatom oder ein salzbildendes Kation bedeutet, W = Sauerstoff und die Reste X, Y und Z keinen Alkylamino-, Hydroxy-oder Hydroxyalkylrest darstellen, auch die Gruppen $COR^6$ oder $SO_2R^7$,

$R^6$ einen $C_1$-$C_8$-Alkylrest, einen $C_1$-$C_8$-Alkoxyrest, einen $C_1$-$C_8$-Alkylthiorest, einen Di-$C_1$-$C_8$-Alkylaminorest, einen $C_1$-$C_6$-Halogenalkylrest, einen gegebenenfalls ein-oder mehrfach durch Halogen, Nitro, $C_1$-$C_4$-Alkyl oder $C_1$-$C_4$-Alkoxy substituierten Phenylrest oder einen gegebenenfalls ein-oder mehrfach durch Halogen, Nitro, $C_1$-$C_4$-Alkyl oder $C_1$-$C_4$-Alkoxy substituierten Phenoxyrest,

$R^7$ einen $C_1$-$C_8$-Alkylrest, einen Trifluormethylrest, einen Trichlormethylrest oder einen gegebenenfalls ein- oder mehrfach durch Halogen, Nitro, $C_1$-$C_4$-Alkyl oder $C_1$-$C_4$-Alkoxy substituierten Phenylrest,

W ein Sauerstoff-oder Schwefelatom,

X ein Wasserstoffatom, eine Nitrogruppe, einen $C_1$-$C_3$-Alkylrest, einen $C_1$-$C_3$-Halogenalkylrest oder einen $C_1$-$C_3$-Alkoxyrest,

Y ein Wasserstoffatom, ein Halogenatom, eine Cyanogruppe, eine Nitrogruppe, einen $C_1$-$C_3$-Alkoxycarbonylrest, einen Pyridylrest, einen N-Methylpyrrolylrest, einen Aminorest, einen $C_1$-$C_4$-Alkylaminorest, einen Di-$C_1$-$C_4$-Alkylaminorest, einen $C_3$-$C_6$-Cycloalkylaminorest, einen Pyrrolidi nylrest, einen Piperidinylrest, einen Aziridinylrest, einen Morpholinylrest, einen $C_1$-$C_4$-Alkylthiorest, einen $C_3$-$C_6$-Cycloalkylthiorest, einen $C_1$-$C_4$-Alkoxyrest, einen $C_3$-$C_6$-Cycloalkoxyrest, einen $C_1$-$C_4$-Alkylsulfonylrest, einen Tolylsulfonylrest, einen $C_3$-$C_6$-Cycloalkylrest, einen gegebenenfalls ein-oder mehrfach durch Halogen, $C_1$-$C_3$-Alkoxy, $C_1$-$C_3$-Alkylthio, Hydroxy, $C_3$-$C_6$-Cycloalkyl, Cyano, Phenyl, Furyl, Tetrahydrofuryl substituierten $C_1$-$C_8$-Alkylrest, der gegebenenfalls durch ein oder mehrere Sauerstoff-oder Schwefelatome unterbrochen ist, einen gegebenenfalls ein- oder mehrfach durch Halogen, $C_1$-$C_3$-Alkyl, $C_1$-$C_3$-Alkoxy, Halogenmethyl oder Nitro substituierten Phenylrest, einen gegebenenfalls ein-oder mehrfach durch Halogen, $C_1$-$C_3$-Alkyl, $C_1$-$C_3$-Alkoxy, Halogenmethyl oder Nitro substituierten Phenoxyrest, einen $C_3$-$C_6$-Alkenylrest oder einen $C_3$-$C_6$-Alkinylrest und

Z ein Wasserstoffatom, einen $C_1$-$C_3$-Alkoxycarbonylrest, einen $C_1$-$C_8$-Alkoxysulfinylrest, einen Phenylsulfonylrest, einen $C_1$-$C_8$-Alkylsulfonylrest, einen $C_1$-$C_3$-Alkylphenylsulfonylrest, einen Carbamoylrest, einen Thiocarbamoylrest, einen Cyanorest, einen Pyridylrest, einen Naphthylrest, einen Acetylrest, einen Benzoylrest, einen Di-$C_1$-$C_6$-alkylcarbamoylrest, einen $C_3$-$C_6$-Cycloalkylcarbamoylrest, einen Amidinorest, einen $C_3$-$C_6$-Cycloalkylrest, einen $C_3$-$C_6$-Alkenylrest, einen $C_3$-$C_6$-Alkinylrest, einen gegebenenfalls ein-oder mehrfach, gleich oder verschieden durch Halogen, Hydroxy, $C_1$-$C_6$-Alkoxy, $C_3$-$C_6$-Cycloalkyl, $C_3$-$C_6$-Cycloalkyloxy, $C_1$-$C_4$-Alkylthio, $C_3$-$C_6$-Cycloalkylthio, Amino, $C_1$-$C_3$-Alkylamino, Di-$C_1$-$C_3$-alkylamino, $C_3$-$C_6$-Cycloalkylamino, Phenyl oder Cyano substituierten $C_1$-$C_8$-Alkylrest, der gegebenenfalls durch ein oder mehrere Sauerstoff-oder Schwefelatome unterbrochen ist, einen gegebenenfalls ein-oder mehrfach, gleich oder verschieden durch Halogen, $C_1$-$C_3$-Alkyl, $C_1$-$C_3$-Alkoxy, Hydroxy,. Cyano oder Nitro substituierten Pyrimidinylrest oder einen gegebenenfalls ein-oder mehrfach, gleich oder ver schieden durch Halogen, $C_1$-$C_3$-Alkyl, $C_1$-$C_3$-Alkoxy, Hydroxy, Cyano oder Nitro substituierten Phenylrest bedeuten, sowie für den Fall, daß W ein Sauerstoffatom und A die Gruppe $COOR^3$, aber $R^3$ nicht ein ungesättigter Alkylrest ist, die N-Oxide der Verbindungen, für den Fall, daß $R^1$ und $R^2$ nicht die gleiche Bedeutung haben, die optischen Isomeren der Verbindungen und für den Fall, daß $R^3$ kein salzbildendes Kation darstellt, die Säureadditionssalze der Verbindungen.

2. Pyrazolo[3,4-b]pyridinderivate nach Anspruch 1, in der

$R^1$ einen $C_1$-$C_4$-Alkylrest,

$R^2$ einen $C_1$-$C_6$-Alkylrest,

A die Gruppen $COOR^3$ oder $CONR^4R^5$,

$R^3$ ein Wasserstoffatom, einen $C_1$-$C_4$-Alkylrest, einen $C_3$-$C_6$-Alkenylrest, $C_3$-$C_6$-Alkinylrest, einen Tetrahydrofurfurylrest oder eine organische Ammoniumverbindung,

$R^4$ ein Wasserstoffatom oder einen $C_1$-$C_4$-Alkylrest,

$R^5$ ein Wasserstoffatom oder einen $C_1$-$C_4$-Alkylrest,

B ein Wasserstoffatom,

W ein Sauerstoffatom,
X ein Wasserstoffatom,
Y ein Wasserstoffatom, einen $C_1$-$C_4$-Alkylrest, einen Halogen-$C_1$-$C_4$-alkylrest, einen $C_1$-$C_4$-Alkoxyrest oder einen $C_1$-$C_4$-Alkylthiorest und
Z einen Methylrest oder einen Phenylrest bedeuten.

3. Verfahren zur Herstellung von Pyrazolo[3,4-b]pyridinderivaten der allgemeinen Formel I, dadurch gekennzeichnet, daß man

A) falls A = COOH und B = Wasserstoff bedeuten, eine Verbindung der allgemeinen Formel II

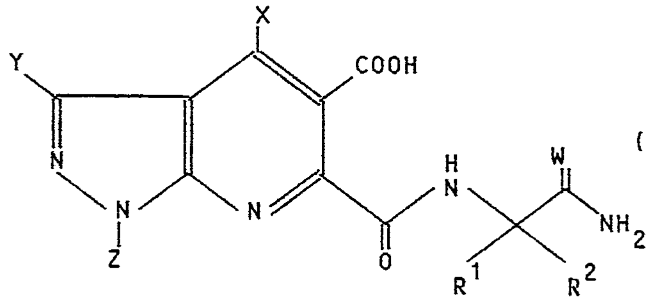

(II) ,

in der $R^1$, $R^2$, W, X, Y und Z die im Anspruch 1 angegebenen Bedeutungen haben, unter alkalischen Bedingungen cyclisiert,

B) falls A = $COOR^3$, $R^3$ aber nicht für ein Wasserstoffatom oder ein salzbildendes Kation steht, und B = Wasserstoff bedeuten, eine Verbindung der allgemeinen Formel III

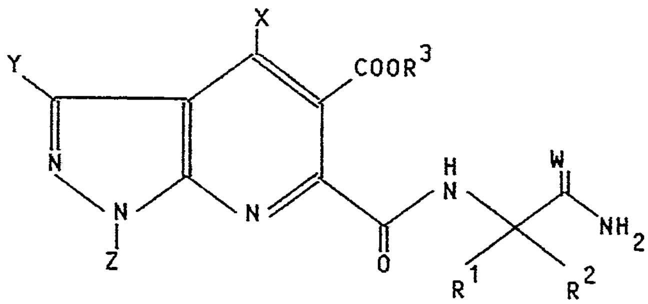

(III) ,

in der $R^1$, $R^2$, $R^3$, W, X, Y und Z die im Anspruch 1 angegebenen Bedeutungen haben, $R^3$ aber nicht für ein Wasserstoffatom oder ein salzbildendes Kation steht, mit einem Gemisch aus Phosphoroxychlorid und Phosphorpentachlorid umsetzt und aus den erhaltenen Hydrochloridsalzen die Ester durch Neutralisation mit einer geeigneten Base in Freiheit setzt,

C) falls A = $COOR^3$, $R^3$ aber nicht für ein Wasserstoffatom oder ein salzbildendes Kation steht, B = Wasserstoff und W = Sauerstoff bedeuten, eine Verbindung der allgemeinen Formel IV

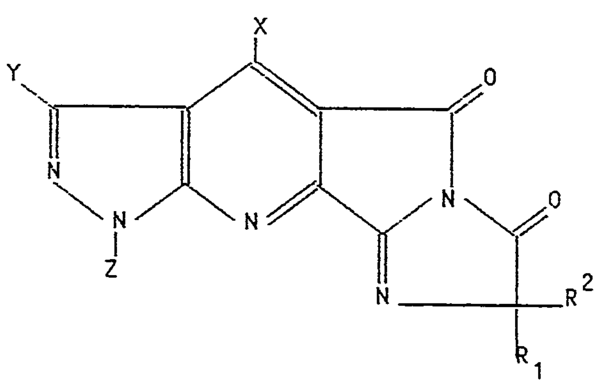

(IV)

in der $R^1$, $R^2$, X, Y und Z die im Anspruch 1 angegebenen Bedeutungen haben, mit einem Alkohol der allgemeinen Formel

$R^3$-OH

und einem entsprechenden Alkalimetallalkoxid der allgemeinen Formel
$R^3$-OM,
in denen $R^3$ die im Anspruch 1 angegebenen Bedeutungen hat, aber nicht für ein Wasserstoffatom oder ein salzbildendes Kation steht, und M ein Alkalimetall darstellt, gegebenenfalls in Gegenwart eines Lösungsmittels umsetzt,

D) falls A = $CONR^4R^5$, B = Wasserstoff und W = Sauerstoff bedeuten, eine Verbindung der allgemeinen Formel IV

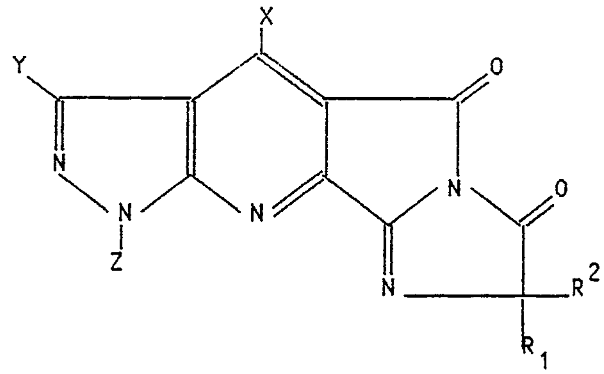

(IV)

in der $R^1$, $R^2$, X, Y und Z die im Anspruch 1 angegebenen Bedeutungen haben, mit einem Amin der allgemeinen Formel
$HNR^4R^5$,
in der $R^4$ und $R^5$ die im Anspruch 1 angegebenen Bedeutungen haben, gegebenenfalls unter Verwendung eines Lösungsmittels umsetzt,

E) falls A = $CH_2OH$, B = Wasserstoff und W = Sauerstoff bedeuten, eine Verbindung der allgemeinen Formel IV

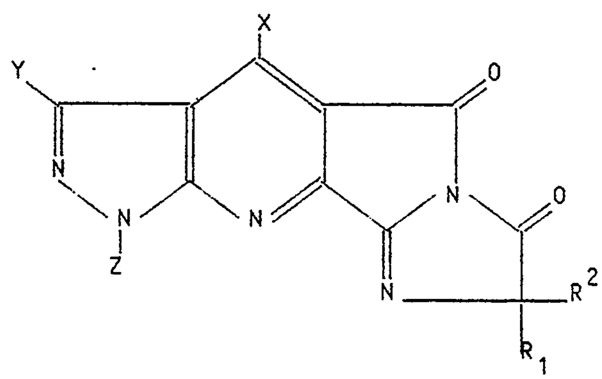

(IV) ,

in der $R^1$, $R^2$, X, Y und Z die im Anspruch 1 angegebenen Bedeutungen haben, mit einem Reduktionsmittel, vorzugsweise Natriumborhydrid, zur Reaktion bringt,

F) falls A = $COOR^3$, $R^3$ = ein salzbildendes Kation und B = Wasserstoff bedeuten, eine Verbindung der allgemeinen Formel Ia

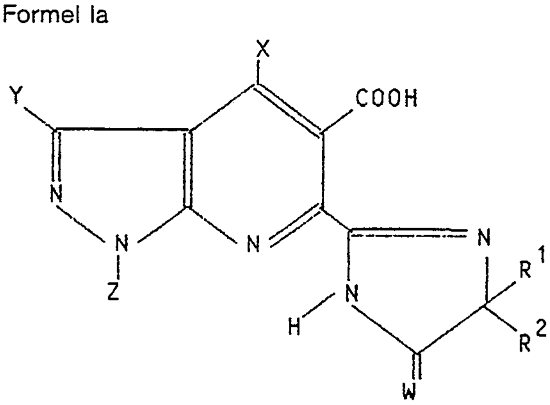

(I a) ,

in der $R^1$, $R^2$, W, X, Y und Z die im Anspruch 1 angegebenen Bedeutungen haben, mit einem Äquivalent einer Base als Salzbildner umsetzt,

G) falls A = $COOR^3$, $R^3$ aber nicht für ein Wasserstoffatom oder ein salzbildendes Kation steht, B = $COR^6$ oder $SO_2R^7$ und W = Sauerstoff bedeuten, eine Verbindung der allgemeinen Formel Ib

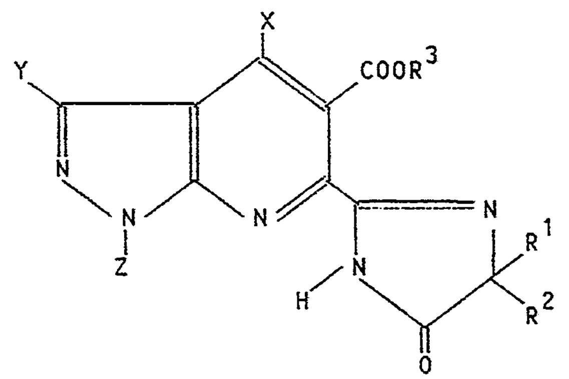

(I b)',

in der $R^1$, $R^2$, $R^3$, X, Y und Z die im Anspruch 1 angegebenen Bedeutungen haben, $R^3$ aber nicht für ein Wasserstoffatom oder ein salzbildendes Kation steht, mit einem Acylhalogenid der allgemeinen Formel

$R^6$-CO - Hal

oder einem Säureanhydrid der allgemeinen Formel

$R^6$-CO - O - CO - $R^6$

oder einem Sulfonylhalogenid der allgemeinen Formel

$R^7$-$SO_2$-Hal,

bei denen $R^6$ und $R^7$ die im Anspruch 1 angegebenen Bedeutungen haben und Hal für ein Halogenatom steht, gegebenenfalls in Gegenwart eines Lösungsmittels und eines säurebindenden Mittels umsetzt oder

H) falls A = $COOR^3$, $R^3$ aber nicht für ein Wasserstoffatom oder ein salzbildendes Kation steht, B = Wasserstoff und W = Sauerstoff bedeuten, eine Verbindung der allgemeinen Formel Ib

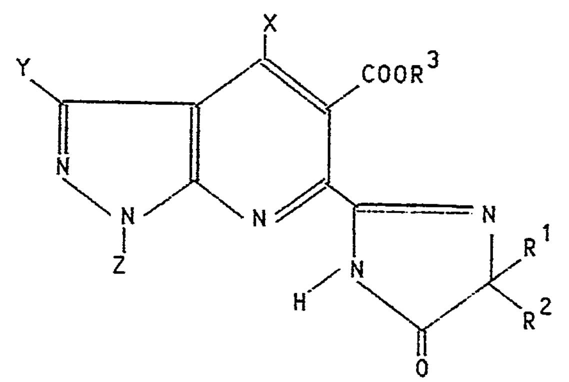

(I b) ,

in der $R^1$, $R^2$, $R^3$, X, Y und Z die im Anspruch 1 angegebenen Bedeutungen haben, $R^3$ aber nicht für ein Wasserstoffatom oder ein salzbildendes Kation steht, mit mindestens einem Äquivalent Halogenwasserstoffsäure in einem organischen Lösungsmittel zu einem Halogenwasserstoffsäure-Additionssalz umsetzt.

4. Mittel mit herbizider Wirkung, gekennzeichnet durch einen Gehalt an mindestens einer Verbindung gemäß den Ansprüchen 1 und 2.

5. Mittel gemäß Anspruch 4, gekennzeichnet durch mindestens eine Verbindung gemäß den Ansprüchen 1 und 2 in Mischung mit Träger-und/oder Hilfsstoffen.

6. Mittel gemäß Anspruch 4, gekennzeichnet durch einen Gehalt an mindestens einer Verbindung hergestellt nach einem Verfahren des Anspruchs 3.

7. Verwendung von Verbindungen gemäß den Ansprüchen 1 und 2 zur Bekämpfung monokotyler und dikotyler Unkrautarten in landwirtschaftlichen Hauptkulturen.